# EUROPEAN PATENT SPECIFICATION

(11) **EP 3 456 818 B1**
(45) Date of publication and mention of the grant of the patent: **20.05.2020**
(21) Application number: 18190029.1
(22) Date of filing: 07.03.2014
(51) Int. Cl.: C12N 5/0783, A61K 35/14

(54) **TARGETING CD138 IN CANCER**
TARGETING VON CD138 BEI KREBS
CIBLAGE CD138 DANS LE DOMAINE DU CANCER

(30) Priority: 07.03.2013 US 201361774040 P
(43) Date of publication of application: 20.03.2019
(62) Divisional of application: 17175815.4
(73) Proprietor: Baylor College of Medicine, Houston, TX 77030-3411 (US)
(72) Inventor: DOTTI, Gianpietro, Chapel Hill, North Carolina 27516 (US); RAMOS, Carlos, A., Houston, TX 77030 (US); SAVOLDO, Barbara, Chapel Hill, North Carolina 27516 (US)
(74) Representative: J A Kemp LLP

(56) References cited:
- WO-A1-2009/080829
- WO-A2-99/60142
- WO-A2-2014/165707
- Laurence Cooper: "T-Cell Immunotherapies for Treating Breast Cancer", , 1 September 2011 (2011-09-01), pages 1-16, XP055126119, Retrieved from the Internet: URL:http://www.dtic.mil/dtic/tr/fulltext/u 2/a554845.pdf [retrieved on 2014-07-01]
- Antonio Di Stasi ET AL: "T Inducible Apoptosis as a Safety Switch for Adoptive Cell Therapy Children's Hospital and Methodist Hospital A bs t r ac t Background", N Engl J Med, 1 January 2011 (2011-01-01), pages 1673-83, XP055123547, Retrieved from the Internet: URL:http://www.nejm.org/doi/pdf/10.1056/NE JMoa1106152 [retrieved on 2014-06-16]
- B. JENA ET AL: "Redirecting T-cell specificity by introducing a tumor-specific chimeric antigen receptor", BLOOD, vol. 116, no. 7, 19 August 2010 (2010-08-19), pages 1035-1044, XP055021403, ISSN: 0006-4971, DOI: 10.1182/blood-2010-01-043737
- Gianpietro Dotti ET AL: "Review Fifteen Years of Gene Therapy Based on Chimeric Antigen Receptors: ''Are We Nearly There Yet?''", , 1 November 2009 (2009-11-01), XP055122213, Retrieved from the Internet: URL:http://online.liebertpub.com/doi/pdf/1 0.1089/hum.2009.142 [retrieved on 2014-06-06]
- JIANG HUA ET AL: "Transfection of chimeric anti-CD138 gene enhances natural killer cell activation and killing of multiple myeloma cells", MOLECULAR ONCOLOGY, vol. 8, no. 2, 12 December 2013 (2013-12-12), pages 297-310, XP028620169, ISSN: 1574-7891, DOI: 10.1016/J.MOLONC.2013.12.001

## Description

### CROSS-REFERENCE TO RELATED APPLICATIONS

This application claims priority to U.S. Provisional Patent Application Serial No. 61/774,040, filed March 7, 2013, which is incorporated by reference herein in its entirety.

### TECHNICAL FIELD

Embodiments of the present disclosure concern at least the fields of cell therapy, immunology, immunotherapy, molecular biology, cell biology, and medicine, including cancer medicine.

### BACKGROUND

In the past 15 years, the use of high-dose chemotherapy and stem-cell rescue, thalidomide, lenalidomide, and bortezomib has prolonged the survival of multiple myeloma (MM) patients (10-year survival is approximately 30%). However, the disease remains essentially incurable. Malignant plasma cells (PC) are sensitive to T-cell immune recognition and elimination as indicated by tumor regression mediated by the graft-versus-MM effects in patients treated with myeloablative or non-myeloablative allogeneic stem cell transplant. This observation has stimulated the development of adoptive T-cell therapies based on the *ex vivo* generation of tumor-specific cytotoxic T lymphocytes (CTLs) in this disease directed to several targetable tumor-associated antigens (TAAs) that are overexpressed in neoplastic PC.

Chimeric antigen receptor (CAR) technology in hematological malignancies has exponentially increased in the past few years. CARs are artificial receptors composed of the single-chain variable fragment (scFv) obtained from a specific monoclonal antibody (antigen-binding site), linked to the intracytoplasmic domains of the CD3ζ chain and costimulatory endodomains. When expressed by T cells, CARs simultaneously mediate MHC-unrestricted antigen recognition and T-cell costimulation.

To target malignant PC using CAR technology, the CD138 molecule (Syndecan-1) that is invariably overexpressed on the cell surface of malignant PC was selected. This target antigen has been validated for MM by successful efforts to develop CD138-specific humanized monoclonal antibodies, that have produced promising preliminary reports in clinical trials. Combining the CD138-antibody specificity with cytotoxicity and longevity of T lymphocytes by using the CAR technology should represent a significant therapeutic advance.

Although CD138 localizes physiologically to baso-lateral surfaces on simple epithelial cells and surrounds stratified epithelial cells administration of CD138-monoclonal antibodies to MM patients has proved safe, likely because the density of CD138 expression on these normal tissues is low. However, expression of CD138-antibody as a CAR on T cells may increase the avidity of the antibody and bring to bear additional T-cell effector mechanisms, thereby causing the "on target" but "off organ" toxicities already observed for other CARs targeting antigens expressed at low levels in normal tissues. This undesired effect can be avoided if CAR-T cells are rapidly eliminated by the activation of an efficient suicide gene or reduced if the CAR is only transiently expressed by T cells, for example after mRNA transfection. However, even if both approaches increase safety, they abrogate the major advantage of adoptive T-cell therapy, which is the long-term immune control of the disease.

The present disclosure satisfies a need in the art to provide effective adoptive T-cell therapy for individuals with CD138-expressing cancer, including at least MM.

### BRIEF SUMMARY

The present disclosure is directed to methods and compositions related to cell therapy. In particular embodiments, the cell therapy is for an individual in need of cell therapy, such as a mammal, including a human. The cell therapy may be suitable for any medical condition, although in specific embodiments the cell therapy is for cancer. The cancer may be of any kind and of any stage. The individual may be of any age or either gender. In specific embodiments, the individual is known to have cancer, is at risk for having cancer, or is suspected of having cancer. The cancer may be a primary or metastatic cancer, and the cancer may be refractory to treatment. The individual may have had a relapse of the cancer. In specific embodiments, the cancer is a hematologic malignancy, such as a B-lineage hematologic malignancy. In some cases, the cancer is not a hematologic malignancy. In some cases the cancer is myeloma, including multiple myeloma. In specific embodiments, the cancer is leukemia, lymphoma, myeloma, breast, lung, brain, colon, kidney, prostate, pancreatic, thyroid, bone, cervical, spleen, anal, esophageal, head and neck, stomach, gall bladder, melanoma, non-small cell lung cancer, and so forth, for example. In particular aspects, the cancer expresses one or more tumor antigens, and in specific embodiments the cell therapy targets the one or more tumor antigens. In particular embodiments, the tumor antigen is CD138 (which may also be referred to as syndecan-1).

In particular embodiments of the disclosure, there are methods and compositions related to cells suitable for use in immunotherapy. In certain aspects, the methods and compositions of the disclosure are an improvement on techniques utilized in the art. In specific cases, embodiments of the disclosure are useful for improvements on cells utilized for immunotherapy of any kind, although in particular cases the immunotherapeutic cells are employed for cancer therapy.

In certain aspects of the disclosure, the individual is provided with cells that provide therapy to the individual. The cells may be of any kind, but in specific embodiments the cells are capable of providing therapy to an individual having cancer cells that express the CD138 antigen. In certain embodiments the cells are provided to an individual known to have a cancer that expresses CD138, such as a B-lineage hematologic malignancy, such as multiple myeloma, although in certain cases the presence of the CD138 antigen is not determined. The cells may be immune cells, such as T-cells. The cells may be cytotoxic T lymphocytes (CTLs), NK-cells, NKT-cells, and so forth, in some cases.

In embodiments of the disclosure, there are methods and compositions related to therapeutic vectors and/or cells that harbor the vectors. In aspects of the disclosure, the therapeutic cells of the disclosure target certain cancer cells comprising the CD138 antigen but do not target other cells having the CD138 antigen, and such dichotomy is the result of environmental-specific expression of the CD138 receptor in the therapeutic cells. In specific embodiments, the methods and compositions of the disclosure concern cells having a CD138-specific chimeric antigen receptor whose expression is under the control of environment-specific regulation. In specific embodiments the environment is hypoxia. In certain embodiments, the methods and compositions of the disclosure concern cells having a CD138-specific chimeric antigen receptor whose expression is under the control of tissue-specific regulation. In some cases, the materials comprise a suicide gene in addition to or alternative to environmental regulation and/or tissue-specific regulation.

In certain aspects of the disclosure, any CAR, including other than CD138, is utilized in the disclosure under hypoxia-specific regulation. Exemplary CARs as an alternative to CD138 or in addition to CD138 include those specific for an antigen selected from the group consisting of Melanoma-associated antigen (MAGE), Preferentially expressed antigen of melanoma (PRAME), survivin, CD19, CD20, CD22, k light chain, CD30, CD33, CD123, CD38, ROR1, ErbB2 ,ErbB3/4, ErbB dimers, EGFr vIII, carcinoembryonic antigen, EGP2, EGP40, mesothelin, TAG72, PSMA, NKG2D ligands, B7-H6, IL-13 receptor a2, MUC1, MUC16, CA9, GD2, GD3, HMW-MAA, CD171, Lewis Y, G250/CAIX, HLA-AI MAGE A1, HLA-A2 NY-ESO-1, PSCA, folate receptor-a, CD44v6, CD44v7/8, aᵥb₆ integrin, 8H9, NCAM, VEGF receptors, 5T4, Foetal AchR, NKG2D ligands, CD44v6, dual antigen, and universal.

In certain aspects to the disclosure, there are immune cells, such as T-cells, that express the CAR.CD138 in the tumor environment, rather than constitutively. The hypoxic MM microenvironment has been demonstrated by direct measurement of the oxygen tension in affected sites (22), and indirectly by the overexpression of HIF-1α (the master regulator of hypoxia-induced responses) (23, 24). One can modulate expression of CAR.CD138 in T cells by controlling its expression with hypoxia response elements, such that engineered T cells express the CAR at functional level only within the hypoxic MM microenvironment, thereby limiting targeting of the antigen in other organs.

In some embodiments of the disclosure, there is an expression vector that encodes a CD138-specific chimeric antigen receptor (CAR) and one or more hypoxia-responsive regulatory elements functionally related thereto. In specific embodiments, the vector further comprises sequence that encodes an inducible suicide gene.

In one embodiment, there is an expression vector that encodes a CD138-specific chimeric antigen receptor (CAR) and that comprises the following: a) one or more hypoxia-responsive regulatory elements that are functionally related to the CD138-specific CAR; and/or b) an inducible suicide gene (caspase 9, herpes simplex virus, thymidine kinase (HSV-tk), cytosine deaminase (CD) and cytochrome P450 are examples). In specific embodiments, the vector is a non-viral vector or a viral vector (such as a retroviral vector, lentiviral vector, adenoviral vector, or adeno-associated viral vector). In specific embodiments, the CD138-specific CAR comprises a IgG1 hinge region. In at least some cases, the CD138-specific CAR comprises an intracellular signaling domain selected from the group consisting of CD28, OX40, 4-1BB, ICOS and a combination thereof. The CD138-specific CAR may comprise a transmembrane domain selected from the group consisting of CD3-zeta and CD28.

Hypoxia-responsive regulatory elements may comprise a VEGF hypoxia-responsive regulatory element, a α1B-adrenergic receptor hypoxia-responsive regulatory element, fatty acid synthase hypoxia-responsive regulatory element, or a combination thereof.

In embodiments of the disclosure, there is a cell comprising a vector of the disclosure. The cell may be a eukaryotic cell, including a human cell. The cells may be autologous, syngeneic, allogeneic, or xenogeneic in relation to a particular individual. In some cases, individuals that are provided methods and/or compositions of the disclosure are in need of cancer treatment, including for a particular type of cancer. In some cases, the individual is in need of treatment for B-lineage hematologic malignancies. In particular cases, the individual is in need of treatment for CD138-expressing cancers, including at least multiple myeloma. The cell may be of any kind, but in specific embodiments the cell is an immune cell, such as a T-cell. The cell may be a cytotoxic T lymphocyte (CTL), natural killer cell, or natural killer T cell, for example. The cell may be an effector cell. The cell may be a T cell that is virus-specific, and the virus may be EBV, CMV, Adenovirus, BK virus, HHV6, RSV, Influenza, Parainfluenza, Bocavirus, Coronavirus, LCMV, Mumps, Measles, Metapneumovirus, Parvovirus B, Rotavirus, West Nile Virus, JC, HHV7, or HIV, for example.

In some cases, the cell comprises at least one other CAR specific for an antigen other than CD138 or as an alternative to CD138. In specific embodiments of the disclosure, the CAR is specific for an antigen selected from the group consisting of Melanoma-associated antigen (MAGE), Preferentially expressed antigen of melanoma (PRAME), survivin, CD19, CD20, CD22, k light chain, CD30, CD33, CD123, CD38, ROR1, ErbB2 ,ErbB3/4, ErbB dimers, EGFr vIII, carcinoembryonic antigen, EGP2, EGP40, mesothelin, TAG72, PSMA, NKG2D ligands, B7-H6, IL-13 receptor a2, MUC1, MUC16, CA9, GD2, GD3, HMW-MAA, CD171, Lewis Y, G250/CAIX, HLA-AI MAGE A1, HLA-A2 NY-ESO-1, PSCA, folate receptor-a, CD44v6, CD44v7/8, aᵥb₆ integrin, 8H9, NCAM, VEGF receptors, 5T4, Foetal AchR, NKG2D ligands, CD44v6, dual antigen, and universal.

In embodiments that are in addition to or as an alternative to using CD138-specific CARs, a cell of the disclosure expresses a secretable engager protein directed to CD138, said protein comprising an activation domain and an antigen recognition domain. The activation domain, antigen recognition domain, or both domains may comprise single chain fragment variable (scFV) antibody moieties, and the activation domain may comprise a scFV that recognizes a molecule selected from the group consisting of CD3, CD16, CD28, CD40, CD134, and CD 137. In particular embodiments, in combination with a CAR there may be an engager encoding a CD138-binding motif combined with a PD-1, CTLA-4, LAG-3, Tim-3, *etc.,* binding or inhibiting motif. In certain embodiments, the antigen recognition domain binds to CD138, although it may bind to Melanoma-associated antigen (MAGE), Preferentially expressed antigen of melanoma (PRAME), survivin, CD19, CD20, CD22, k light chain, CD30, CD33, CD123, CD38, ROR1, ErbB2 ,ErbB3/4, ErbB dimers, EGFr vIII, carcinoembryonic antigen, EGP2, EGP40, mesothelin, TAG72, PSMA, NKG2D ligands, B7-H6, IL-13 receptor a2, MUC1, MUC16, CA9, GD2, GD3, HMW-MAA, CD171, Lewis Y, G250/CAIX, HLA-AI MAGE A1, HLA-A2 NY-ESO-1, PSCA, folate receptor-a, CD44v6, CD44v7/8, aᵥb₆ integrin, 8H9, NCAM, VEGF receptors, 5T4, Foetal AchR, NKG2D ligands, CD44v6, dual antigen, or universal tumor antigens.

In embodiments of the disclosure, there is a method of treating cancer in an individual, comprising the step of delivering to the individual a therapeutically effective amount of a vector of the disclosure. In certain embodiments, there is a method of treating cancer in an individual, comprising the step of delivering to the individual a therapeutically effective amount of cells of the disclosure.

In embodiments of the disclosure, there is an expression vector that encodes a tumor antigen-specific CAR and one or more hypoxia-responsive regulatory elements functionally related thereto. The vector may further comprise sequence that encodes an inducible suicide gene. In embodiments of the disclosure, there is an expression vector that encodes a tumor antigen-specific CAR and that comprises the following: a) one or more hypoxia-responsive regulatory elements that are functionally related to the tumor antigen-specific CAR; and/or b) an inducible suicide gene. Any vector of the disclosure may include a CAR specific for a tumor antigen selected from the group consisting of Melanoma-associated antigen (MAGE), Preferentially expressed antigen of melanoma (PRAME), survivin, CD19, CD20, CD22, k light chain, CD30, CD33, CD123, CD38, ROR1, ErbB2 ,ErbB3/4, ErbB dimers, EGFr vIII, carcinoembryonic antigen, EGP2, EGP40, mesothelin, TAG72, PSMA, NKG2D ligands, B7-H6, IL-13 receptor a2, MUC1, MUC16, CA9, GD2, GD3, HMW-MAA, CD171, Lewis Y, G250/CAIX, HLA-AI MAGE A1, HLA-A2 NY-ESO-1, PSCA, folate receptor-a, CD44v6, CD44v7/8, aᵥb₆ integrin, 8H9, NCAM, VEGF receptors, 5T4, Foetal AchR, NKG2D ligands, CD44v6, dual antigen, and universal. Methods of treating cancer with these vectors are encompassed in the disclosure. Cells expressing these vectors and methods of using the cells to treat cancer are also encompassed in the disclosure.

In embodiments of the disclosure, there is a kit comprising a vector as described herein and/or cells as described herein.

In embodiments of the invention, there is a polynucleotide that encodes at least one CD138-specific recognition moiety and has one or more hypoxia-responsive regulatory elements functionally related thereto. In a specific embodiment, the CD138-specific recognition moiety comprises a CD138-specific chimeric antigen receptor (CAR), a CD138-specific engager molecule, or both. In specific embodiments, the polynucleotide is further defined as an expression vector. In certain embodiments, the polynucleotide further comprises sequence that encodes an inducible suicide gene. In some cases, the vector is a non-viral vector or a viral vector, including a retroviral vector, lentiviral vector, adenoviral vector, or adeno-associated viral vector. In certain embodiments, the CD138-specific CAR comprises a IgG1 hinge region. In some cases, the CD138-specific CAR comprises an intracellular signaling domain selected from the group consisting of CD28, OX40, 4-1BB, ICOS and a combination thereof. In particular cases, the CD138-specific CAR comprises a transmembrane domain selected from the group consisting of CD3-zeta and CD28. In particular aspects, the suicide gene is selected from the group consisting of caspase 9, herpes simplex virus, thymidine kinase (HSV-tk), cytosine deaminase (CD) and cytochrome P450. In some embodimetns, the hypoxia-responsive regulatory element comprises a VEGF hypoxia-responsive regulatory element, a α1B-adrenergic receptor hypoxia-responsive regulatory element, fatty acid synthase hypoxia-responsive regulatory element, or a combination thereof.

In one embodiment, there is provided a cell that comprises a polynucleotide of the disclosure. In specific embodimetns, the cell is a eukaryotic cell, such as a human cell, including an immune cell. In certain embodiments, the cell is further defined as autologous, syngeneic, allogeneic, or xenogeneic in relation to a particular individual. The individual may be in need of cancer treatment, including at least for B-lineage hematologic malignancies such as multiple myeloma. In some cases, the cell is further defined as a cytotoxic T lymphocyte (CTL), natural killer cell, or natural killer T cell. The cell may comprise native receptors specific for virus latency proteins. Exemplary viruses include EBV, CMV, Adenovirus, BK virus, HHV6, RSV, Influenza, Parainfluenza, Bocavirus, Coronavirus, LCMV, Mumps, Measles, Metapneumovirus, Parvovirus B, Rotavirus, West Nile Virus, JC, HHV7, or HIV. In particular embodiments, the cell comprises at least one other CAR specific for an antigen other than CD138. The CAR may be specific for an antigen selected from the group consisting of Melanoma-associated antigen (MAGE), Preferentially expressed antigen of melanoma (PRAME), survivin, CD19, CD20, CD22, k light chain, CD30, CD33, CD123, CD38, ROR1, ErbB2 ,ErbB3/4, ErbB dimers, EGFr vIII, carcinoembryonic antigen, EGP2, EGP40, mesothelin, TAG72, PSMA, NKG2D ligands, B7-H6, IL-13 receptor a2, MUC1, MUC16, CA9, GD2, GD3, HMW-MAA, CD171, Lewis Y, G250/CAIX, HLA-AI MAGE A1, HLA-A2 NY-ESO-1, PSCA, folate receptor-a, CD44v6, CD44v7/8, aᵥb₆ integrin, 8H9, NCAM, VEGF receptors, 5T4, Foetal AchR, NKG2D ligands, CD44v6, dual antigen, and universal. In particular embodiments, the CD138-specific recognition moiety comprises a CD138-specific engager molecule having an activation domain and an antigen recognition domain, wherein both domains comprise single chain fragment variable (scFV) antibody moieties. In some cases, the activation domain is a scFV that recognizes a molecule selected from the group consisting of CD3, CD16, CD28, CD40, CD134, and CD137.

In one embodiment, there is amethod of treating cancer in an individual, comprising the step of delivering to the individual a therapeutically effective amount of a polynucleotide of the disclosure. In some embodiments, there is a method of treating cancer in an individual, comprising the step of delivering to the individual a therapeutically effective amount of cells of the disclosure. In particular embodiments, there is a kit comprising a vector of the disclosure. In specific embodiments, there is a kit comprising one or more cells of the disclosure. In some aspects, there is an expression vector that encodes a tumor antigen-specific CAR and one or more hypoxia-responsive regulatory elements functionally related thereto. In certain embodiments, the vector further comprises sequence that encodes an inducible suicide gene. The vector may comprise a tumor antigen selected from the group consisting of Melanoma-associated antigen (MAGE), Preferentially expressed antigen of melanoma (PRAME), survivin, CD19, CD20, CD22, k light chain, CD30, CD33, CD123, CD38, ROR1, ErbB2 ,ErbB3/4, ErbB dimers, EGFr vIII, carcinoembryonic antigen, EGP2, EGP40, mesothelin, TAG72, PSMA, NKG2D ligands, B7-H6, IL-13 receptor a2, MUC1, MUC16, CA9, GD2, GD3, HMW-MAA, CD171, Lewis Y, G250/CAIX, HLA-AI MAGE A1, HLA-A2 NY-ESO-1, PSCA, folate receptor-a, CD44v6, CD44v7/8, aᵥb₆ integrin, 8H9, NCAM, VEGF receptors, 5T4, Foetal AchR, NKG2D ligands, CD44v6, dual antigen, and/or universal.

The foregoing has outlined rather broadly the features and technical advantages of the present invention in order that the detailed description of the invention that follows may be better understood. Additional features and advantages of the invention will be described hereinafter which form the subject of the claims of the invention. It should be appreciated by those skilled in the art that the conception and specific embodiment disclosed may be readily utilized as a basis for modifying or designing other structures for carrying out the same purposes of the present invention. It should also be realized by those skilled in the art that such equivalent constructions do not depart from the spirit and scope of the invention as set forth in the appended claims. The novel features which are believed to be characteristic of the invention, both as to its organization and method of operation, together with further objects and advantages will be better understood from the following description when considered in connection with the accompanying figures. It is to be expressly understood, however, that each of the figures is provided for the purpose of illustration and description only and is not intended as a definition of the limits of the present invention.

### BRIEF DESCRIPTION OF THE DRAWINGS

For a more complete understanding of the present invention, reference is now made to the following descriptions taken in conjunction with the accompanying drawing, in which:

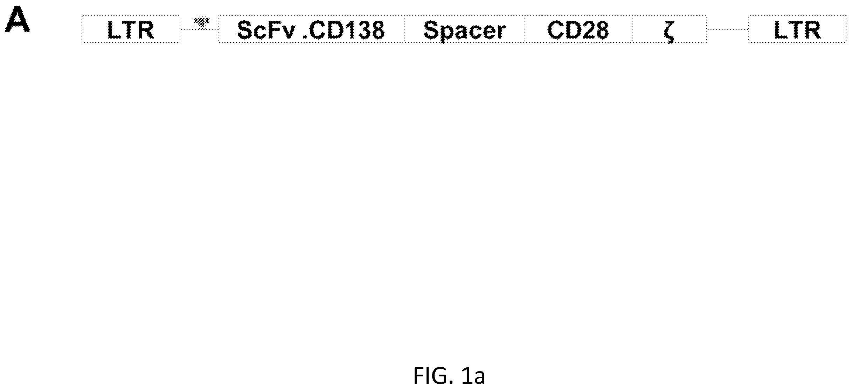

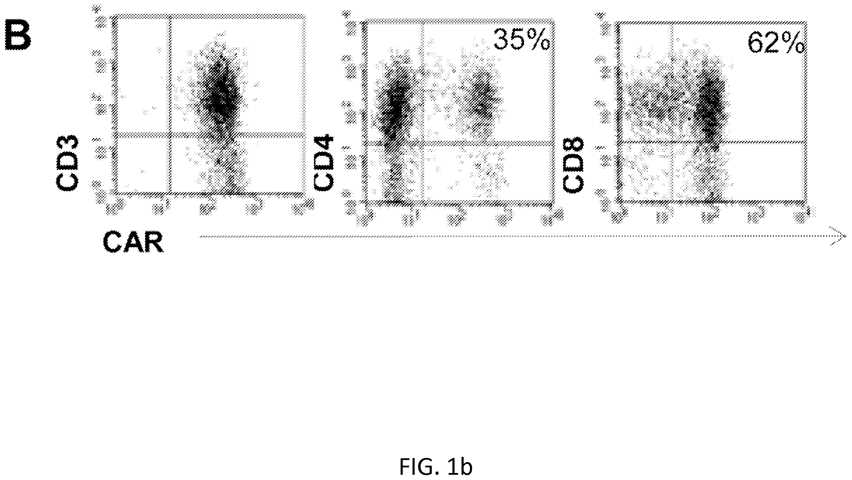

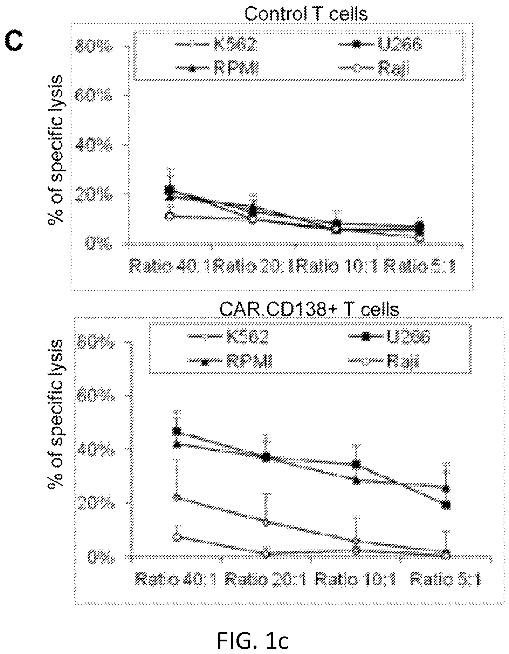

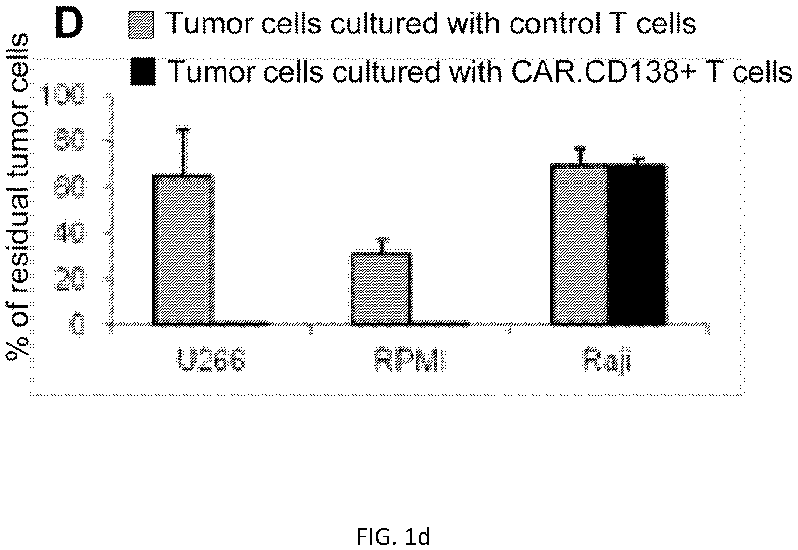

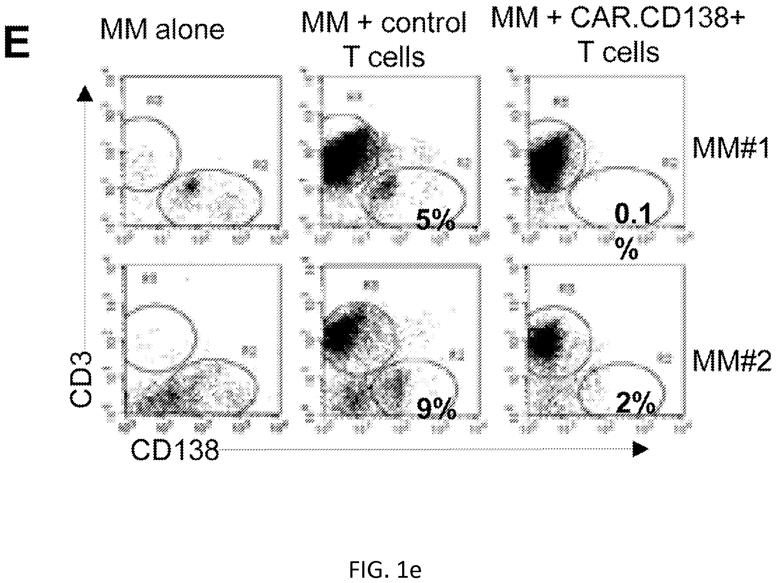

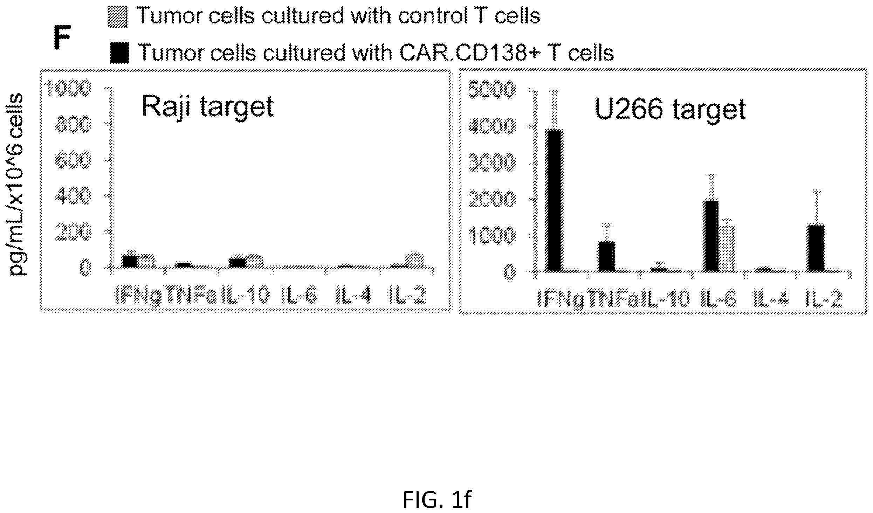

FIG. 1. Generation and characterization of the CAR.CD138. Panel A. Schematic representation of the CAR.CD138 encoded in a retroviral vector. Panel B. After retroviral transduction the CAR is efficiently expressed in activated CD4+ and CD8+ T cells. Panel C illustrates the cytotoxic activity evaluated with a standard 51Cr release assay of control and CAR.CD138 T cells against CD138+ targets (RPMI and U266), CD138- target (Raji) and NK target (K562). Data represent the mean ± SD of 4 different donors. Panel D. Control and CAR+ T cells were co-cultured with CD138+ targets (U266 and RPMI) or CD138- targets (Raji) (ratio T cells:tumor cells 5:1). After 4 days of culture, cells were collected and stained with CD3 and CD138 to evaluate the growth of tumor cells. No tumor cells were detectable when they were cocultured with CAR+ T cells. Data represent the mean ± SD of 4 different donors. Panel E shows the coculture experiments using BM samples containing CD138+ malignant PC from 2 donors. CAR+ T cells but not control cells eliminate malignant PC. Panel F illustrates the Th1 profile of T cells in response to the CD138+ target U266 cells.

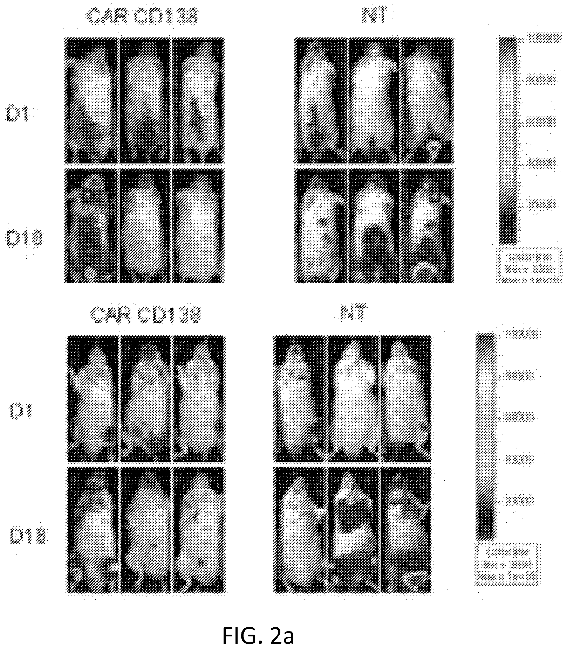

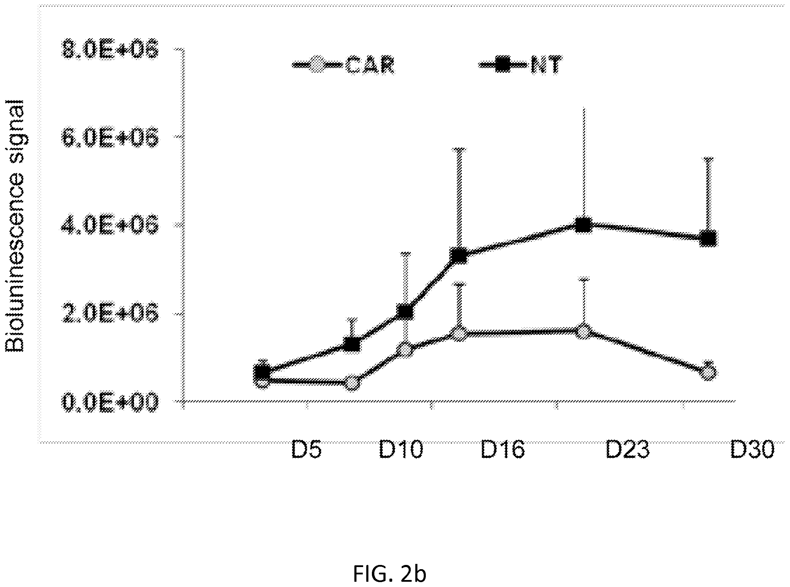

FIGS. 2a and 2b. CAR.CD138+ T lymphocytes have anti-MM effect *in vivo.* Control and CAR.CD138+ T cells were infused i.v. in SCID mice bearing U266 cells labeled with FFLuc. No exogenous cytokines were used. Tumor growth was monitored using an *in vivo* imaging system (Xenogen-IVIS Imaging System). Left panels illustrate that the tumor growth measured as intensity of the signal (p/s/cm2/sr) was significantly higher in mice receiving control versus CAR.CD138+ T cells. The right panel illustrates the average ± SD of 5 mice per group in two different experiments.

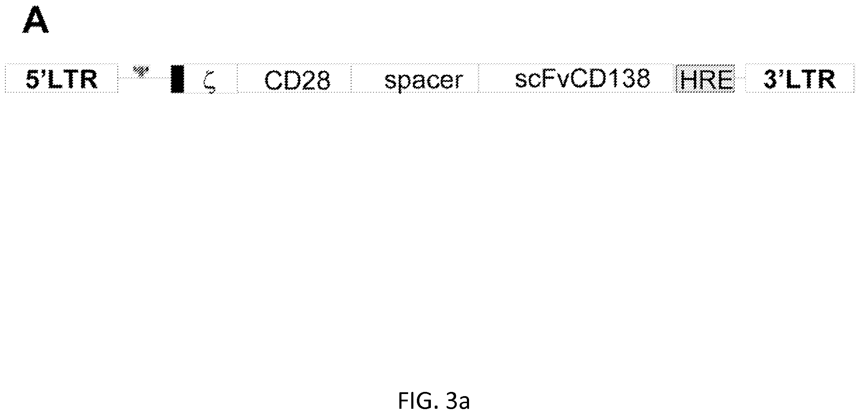

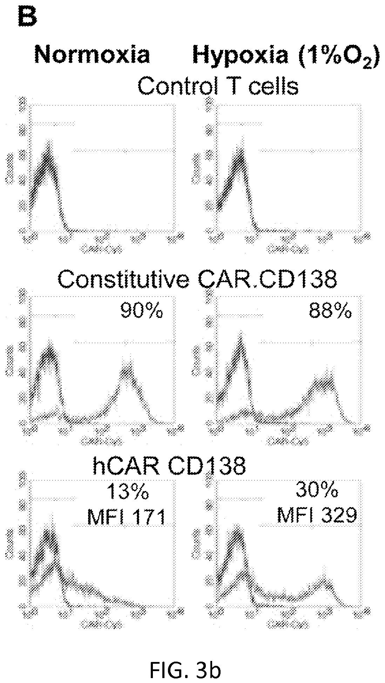

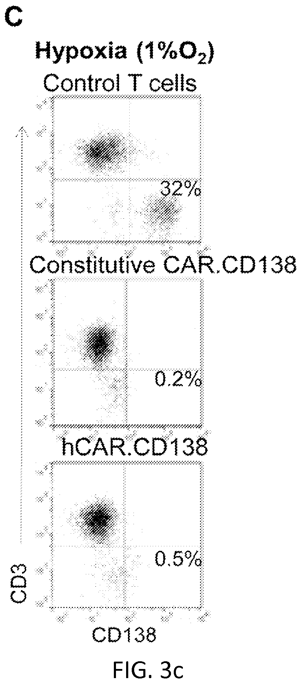

FIG. 3. Generation and characterization of the hCAR.CD138. Panel A. Schematic representation of the hCAR.CD138 encoded in a retroviral vector. Panel B. Expression of the constitutive CAR.CD138 and hCAR.CD138 in T cells in hypoxia for 48 hours as compared with normoxia. Panel C. Control, constitutive CAR.CD138+ and hCAR.CD138+ T cells were co-cultured with CD138+ targets (U266) in hypoxia (ratio T cells:tumor cells 2:1). After 3 days of culture, cells were collected and stained with CD3 and CD138 to evaluate the growth of tumor cells. No tumor cells were detectable when these cells were cocultured with T cells expressing the constitutive CAR.CD138 or hCAR.CD138.

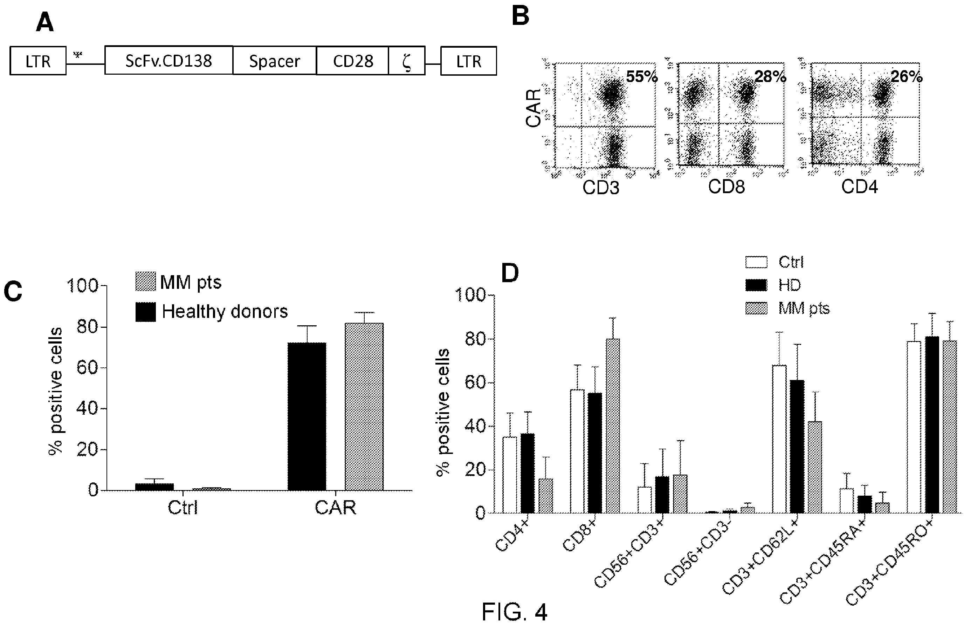

FIG. 4. CAR.CD138 can be efficiently and stably expressed in T cells from both healthy donors and multiple myeloma (MM) samples. (A) Retroviral vector encoding the CD138-specific CAR incorporating the CD28 endodomain. (B) Expression of CAR.CD138 was detected on both CD8⁺ and CD4⁺ T cells. (C) The transduction efficiency of CAR.CD138 was 74%±9% in the 7 different lines generated from healthy donors and 82%±5% in 6 lines generated from MM patients. (D) Both control and transduced T cells from healthy donors contained a balanced proportion of CD3⁺CD8⁺ T cells (57%±26% and 54%±14%) and CD3⁺CD4⁺ T cells (35%±17% and 37%±13%), while T cells from MM patients were more skewed to contain CD8⁺ cells (80%±10%). Less than 2% of the cells were CD3⁻CD56⁺CD16⁺. Transduced T cells from healthy donors and MM patients contained also a proportion of memory and effector memory cells (CD45RO⁺: 82%±16% and 79%±9%, respectively; CD62L⁺=51%±17% and 42%±14%, respectively) compatible with the *ex vivo* expansion procedure.

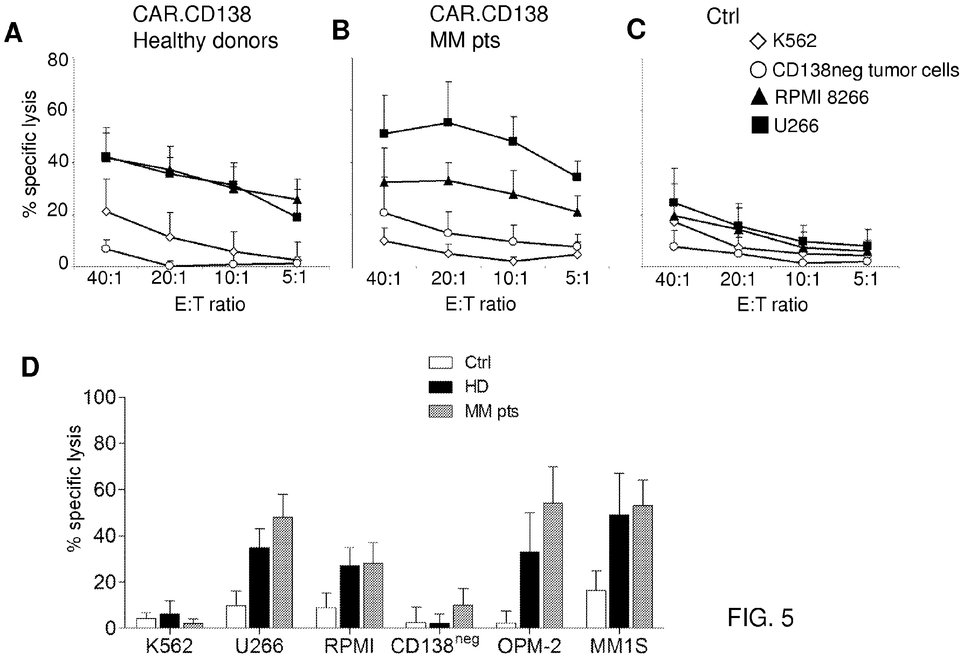

FIG. 5 CAR.CD138⁺ T cells target CD138⁺ tumor cell lines. T cells from healthy donors expressing CAR.CD138 lyzed CD138⁺ MM-derived cell lines U266, RPMI-8266, OPM-2 and MM.1S at a significantly higher rate (31%±8%, 30%±8%, 39%±7% and 65%±13% at 10:1 E:T ratio, respectively) as compared to control T cells (9%±2%, 7%±6%, 2%±6% and 5%±2% at 10:1 E:T ratio, respectively) in a standard 51Cr release assay (A,D). Similar pattern of killing was observed when transduced T cells were generated form MM patients (B). In contrast, CAR.CD138⁺ T cells had negligible activity against CD138⁻ targets (Raji, ARH-77 and K562) (A,B,D). Negligible killing was also observed with control T cells (C). (D) Illustrates the summary ± SD of at least 5 independent experiments.

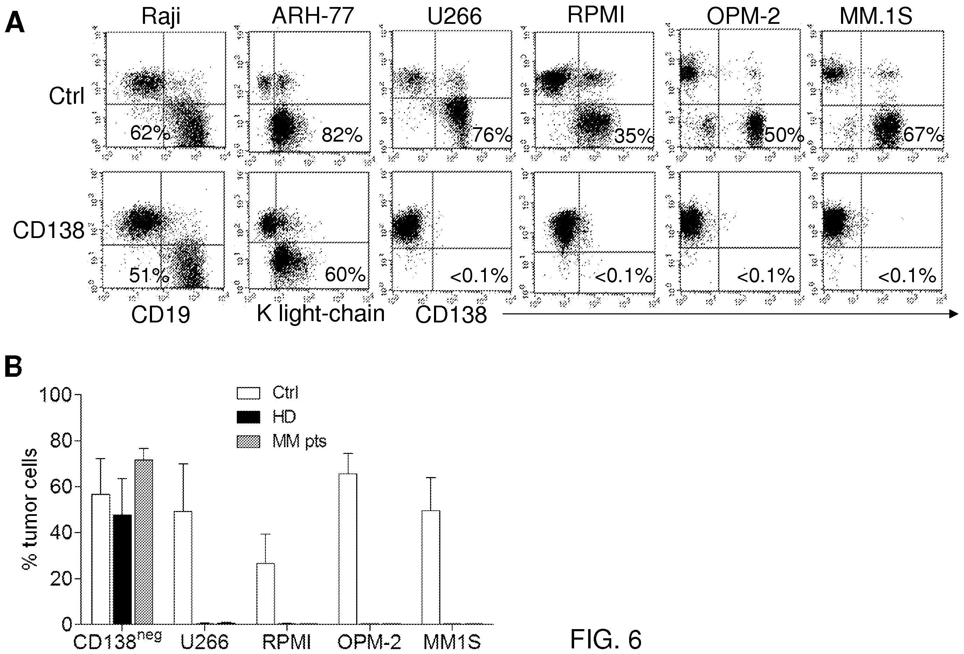

FIG. 6. CAR.CD138⁺ T cells eliminate CD138⁺ tumor cells in co-culture experiments. (A) To evaluate the long-term ability of CAR.CD138⁺ to eliminate CD138⁺ tumor cells, CAR⁺ or control T cells were co-cultured with CD138⁺ (U266, RPMI-8266, OPM-2 and MM.1S) or CD138⁻ (Raji or ARH77) tumor cells in the absence of exogenenous cytokines, and enumerated residual tumor cells after 5-7 days by FACS analysis. In the presence of CAR⁺ T cells there was complete elimination of CD138⁺ tumors, while tumor cells overgrew in cultures with control T cells (U266: 57%±18%; RPMI: 29%±13%; OPM-2: 63%±13%; MM.1S: 67%±1%;). In contrast, lack of antitumor effects against CD138⁻ target cells was observed. (B) Illustrates the summary ± SD of 5 independent experiments.

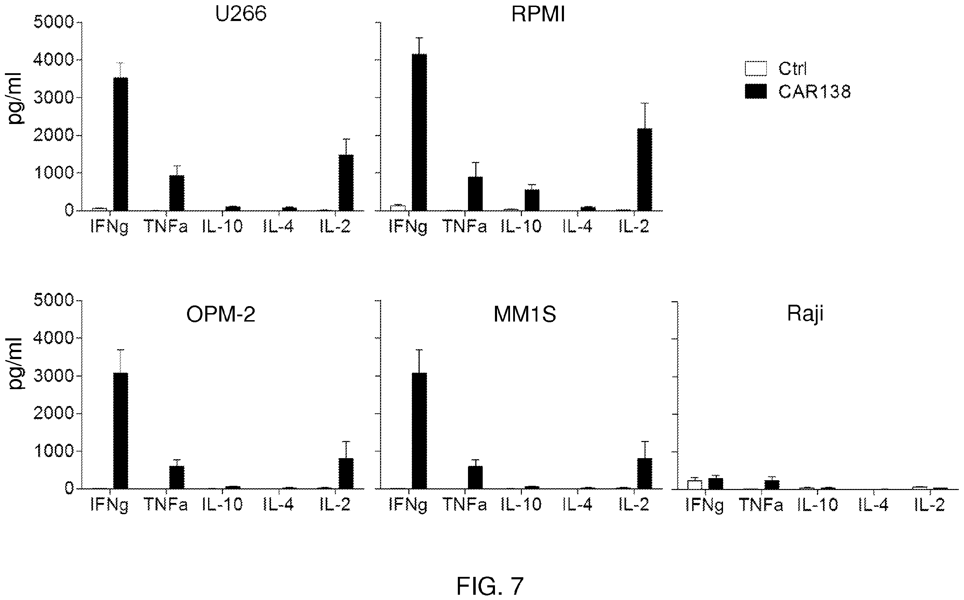

FIG. 7. CAR.CD138⁺ T cells show a Th1 profile in response to tumor cells. To evaluate the cytokine profile of CAR.CD138⁺, CAR⁺ or control T cells were co-cultured with CD138⁺ (U266, RPMI-8266, OPM-2 and MM.1S) or CD138⁻ (Raji) tumor cells. Cuture supernatant were collected after 24 hours and analysed for the presence of Th1 and Th1 cytokines.

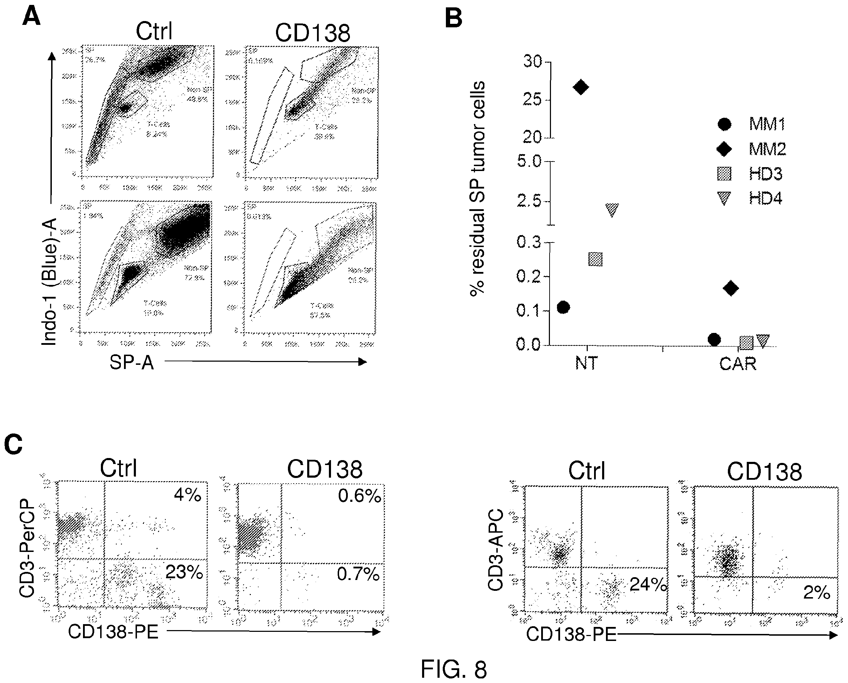

FIG. 8. CAR.CD138⁺ T cells target putative cancer stem cells. To ensure that the approach would also target putative cancer stem cells, expression of CD138 was studied by SP cells contained in the RPMI-8266 tumor cell cells and then monitored if this subset could also be effectively eliminated by CAR⁺ T cells. In co-cultures with control T cells, not only RPMI-8266 cells were still present (73%±7%), but also an average of 6% (range from 0.11% to 26.7%) of SP cells was still present (A,B). In contrast, in cultures with CAR⁺ T cells, RPMI cells were significantly reduced (11%±10%) and no SP cells (0.04%±0.07%) were detectable (A,B). To further confirm this capability, SP cells were directly sorted from the RPMI cell line and cultured with control or CAR⁺ T cells (C). SP cells sorted cells were completely eliminated only in the presence of transduced T cells. Two representative co-culture experiments are shown.

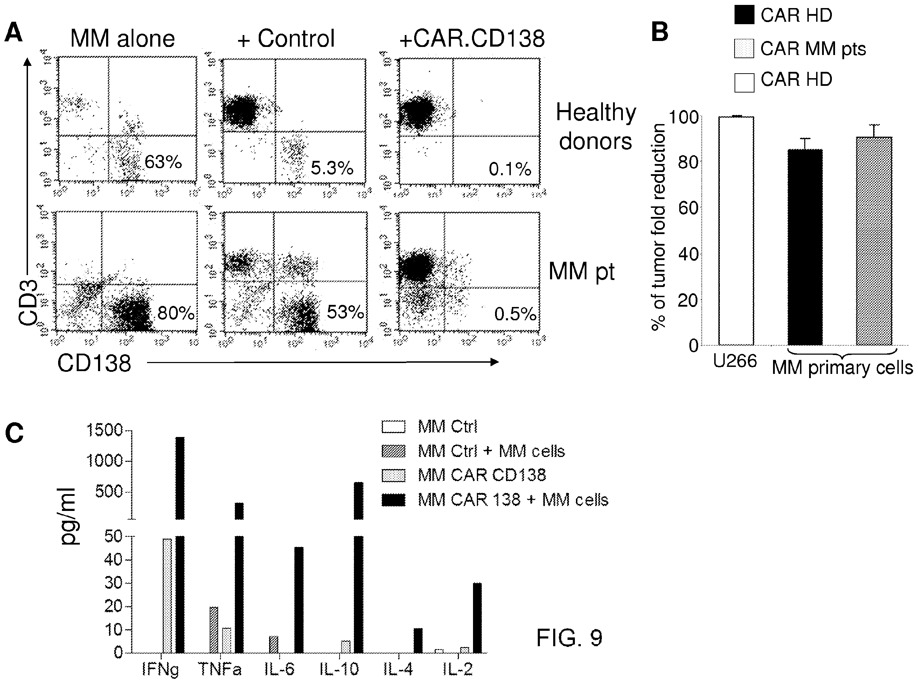

FIG. 9. CAR.CD138⁺ T cells target primary myeloma cells. (A) CAR⁺ T cells generated from healthy donors successfully eliminated CD138 selected tumor cells from MM patients in contrast to control T cells (<80% fold reduction). (B) Similarly autologous CAR⁺ T cells eliminated primary MM cells (37%±14%) as compared to control T cells (2%±2%) (90%±10% fold reduction). (C) Cytokine profile in these experiments was consistent with Th1.

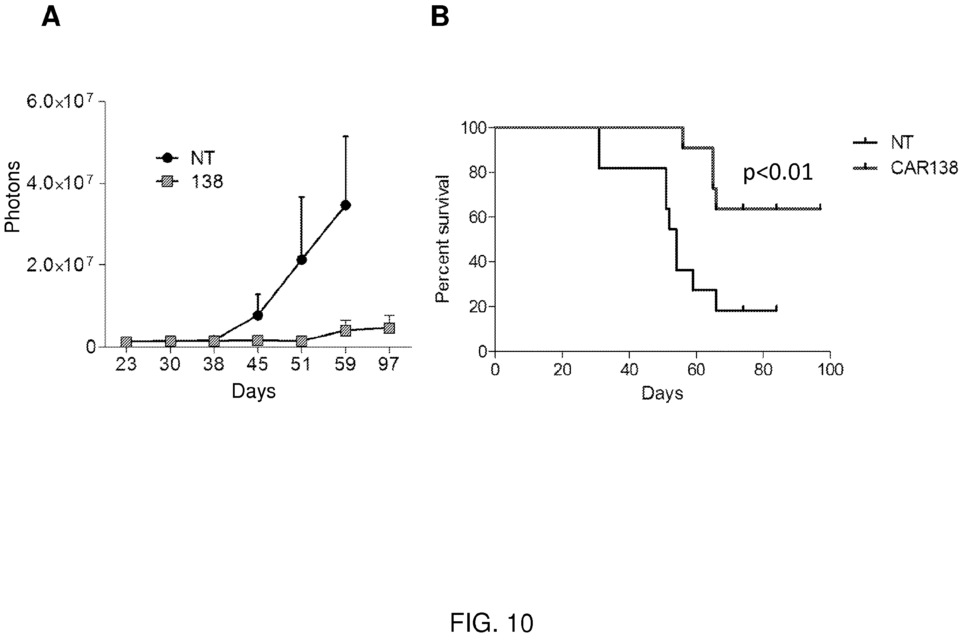

FIG. 10. CAR.CD138⁺ T cells have antitumor activity *in vivo.* NSG mice received intravenous administration of 4x10⁶ FireFlyLuciferase labeled OPM-2 cells, followed by 3 i.v. infusions with CAR.CD138⁺ T cell infusions (1x10⁷). Cioluminescent imaging (BLI) was performed starting on day 23 to monitor tumor growth. (A) Average photons/sec/cm²/sr per mouse, determined by BLI, comparing mice treated with control T cells (NT, n=11, black circles) or CAR.CD138⁺ T cells (n=11, gray squares). Mean ± SEM, *p=0.05 on day 59. Summary of 3 independent experiments. (D) Kaplan-Meier survival curve of mice treated with CAR.CD138⁺ T cells or control T cells (p<0.01).

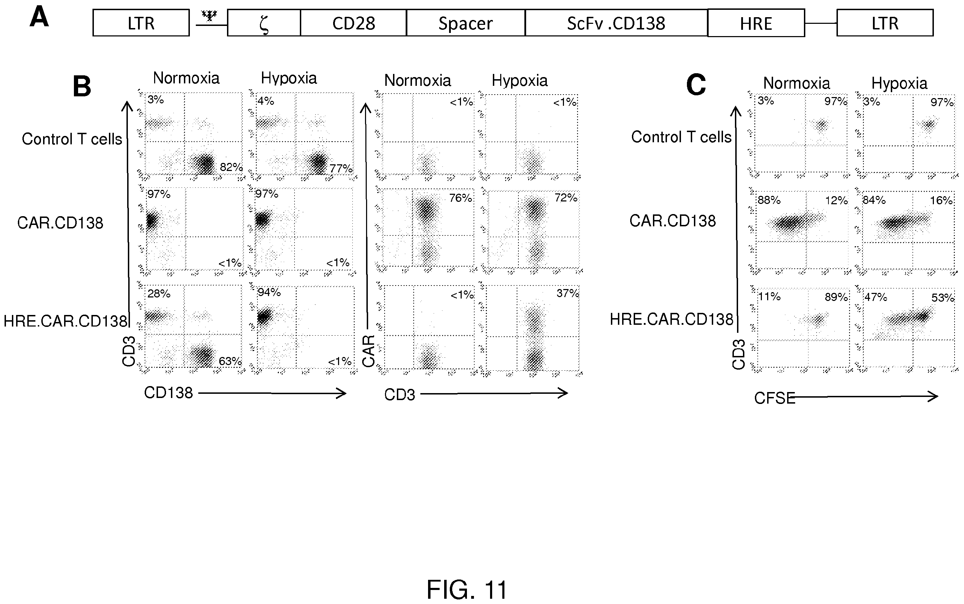

FIG. 11. Generation and function of hypoxia inducible CAR.CD138 (HRE.CAR.CD138). (A) Schematic representation of the HRE.CAR.CD138 encoded in a retroviral vector. (B) Control, constitutive CAR.CD138⁺ or HRE.CAR.CD138⁺ T cells were co-cultured with the CD138⁺ targets (U266) in normoxia (20% O₂ tension) or hypoxia (1% O₂ tension). After 4 days of culture, cells were collected and stained with CD3 and CD138 to evaluate the growth of tumor cells. Expression of CAR on T cells was also evaluated. HRE.CAR.CD138⁺ T cells eliminated the tumor cells in hypoxic conditions. (C) Control, constitutive CAR.CD138⁺ or HRE.CAR.CD138⁺ T cells were labelled with CSFE and co-cultured with the CD138⁺ targets (U266) in normoxia (20% O₂ tension) or hypoxia (1% O₂ tension). After 4 days of culture, cells were collected, stained with CD3 and dilution of CSFE measured by flow cytometry. HRE.CAR.CD138⁺ T cells proliferated in hypoxic conditions.

### DETAILED DESCRIPTION

In keeping with long-standing patent law convention, the words "a" and "an" when used in the present specification in concert with the word comprising, including the claims, denote "one or more." Some embodiments of the disclosure may consist of or consist essentially of one or more elements, method steps, and/or methods of the disclosure. It is contemplated that any method or composition described herein can be implemented with respect to any other method or composition described herein.

The terms "hypoxic" as used herein refers to 20% O₂ tension and "hypoxia" as used herein refers to 1% O₂ tension. In specific embodiments, physiological tissue hypoxia is considered <∼60mmHg.

### I. General Embodiments

In embodiments of the disclosure, there are methods and compositions for treating CD138-expressing cancer cells. In specific embodiments, the cancer is haematological malignancies, such as multiple myeloma (MM), or solid tumors, such as breast cancer, and solid tumors that are almost invariantly hypoxic. The methods and compositions are related to providing treatment that delivers therapy to certain tissues or cells in need but that avoids delivery to CD138-expressing non-cancerous cells. The methods and compositions are related to providing treatment that delivers therapy to cancerous tissues or cells expressing CD138 but that avoids delivery to cells that express CD138 but are not in need of cancer therapy. In particular embodiments the therapy is effective in tissues or environments that are hypoxic and the therapy is ineffective in tissues or environments that are normoxic. In aspects to the disclosure, the therapy is effective in tissues or environments that are hypoxic and is not effective in normoxic tissues or environments because a therapeutic moiety is not present in normoxic tissues or environments. In some cases, cell therapy may be effective in tissues or environments that are hypoxic and is not effective in normoxic tissues or environments because the cells lack expression of a therapeutic moiety in normoxic tissues or environments but that is present in hypoxic tissues or environments. The therapeutic moiety comprises at least CD138 antigen-specific CAR, in embodiments of the disclosure. Other tissue-specific antigens may be targeted with corresponding moieties also having tissue-specific or environment-specific expression.

Particular aspects of the disclosure provide therapy for MM for an individual known to have MM, suspected of having MM, or at risk for developing MM. The individual may be determined to have MM by means other than identification of CD138-positive cancer cells, in some cases. In particular embodiments therapy for MM has already been provided or is being provided to the individual. The individual may be refractory to one or more MM therapies (other than the disclosure) of any kind initially or after some period of time on the therapy.

Particular aspects of the disclosure provide therapy for breast cancer for an individual known to have it, suspected of having it, or at risk for developing it (such as having one or more indicative genetic markers or a family or personal history). The individual may be determined to have breast cancer by means other than identification of CD138-positive cancer cells, in some cases. In particular embodiments therapy for breast cancer has already been provided or is being provided to the individual. The individual may be refractory to one or more breast cancer therapies (other than the disclosure) of any kind initially or after some period of time on the therapy.

Despite significant improvements in the therapeutic options for MM, the disease remains essentially incurable. In particular, the inventors and others have shown that T cells expressing chimeric antigen receptors (CARs) targeted to tumor-associated antigens (TAAs) and incorporating adequate co-stimulatory endodomains are an attractive means of treating B-lineage hematologic malignancies. Because malignant plasma cells (PC) express high levels of the CD138 antigen, it was reasoned that they could be effectively eliminated by T cells redirected to target this antigen. Indeed, a CD138-specific CAR (CAR.CD138) has shown useful activity against CD138+ tumor cells in initial studies. One caveat to the use of CD138 is that the antigen is also expressed at low levels on the basolateral surfaces of epithelial cells, mesenchymal cells, vascular smooth muscle cells, endothelial cells and neural cells, for example, increasing the risk of "on target" but "off organ" or "off tissue" toxicity with CAR-modified T cells. Thus, in specific embodiments of the disclosure one can exploit the hypoxic nature of the normal and MM bone marrow (BM) microenvironment by expressing the CAR.CD138 under the inducible control of hypoxia-responsive elements. One can also define the most advantageous immune elements for co-stimulation of CAR-modified T cells in a hypoxic environment both *in vitro* and *in vivo,* such as in a xenogenic mouse model. Finally, to further increase the safety of embodiments of the disclosure, one can incorporate within a construct a suicide gene, such as a previously validated suicide gene based on inducible caspase9 (iC9). This strategy allows the rapid ablation of CAR-modified T cells should larger-than-anticipated numbers escape the hypoxic BM environment and retain sufficient functional CAR expression to cause "on-target" but "off-organ" toxicity. In certain aspects, one can manufacture the clinical grade retroviral vector and CAR-modified autologous T cell lines from MM patients and infuse these cells into patients with relapsed MM enrolled in a Phase I clinical trial. In certain cases one can evaluate the safety of the procedure, the *in vivo* survival of the infused T cells, and the accumulation of these cells in the BM and the differential expression of the CAR by T cells in peripheral blood (PB) versus BM. If toxicity occurs, one can determine the activity of the iC9 safety gene *in vivo.* One can also assess whether T-cell infusions provide disease control in patients with detectable disease.

It is an object of the disclosure to exploit the hypoxic nature of MM BM microenvironment by expressing the CAR.CD 138 under the inducible control of hypoxia-responsive elements (HRE). One can also define the most advantageous immune elements for costimulation of CAR-modified T cells in a hypoxic environment both *in vitro* and *in vivo* in a xenogenic mouse model. Finally, to further increase the safety of the proposed approach, one can incorporate within the construct a previously validated suicide gene based on inducible caspase9 (iC9). In certain embodiments, inducible caspase 9 (iCaspase9) is dimerizable using a small molecule, *e.g.,* AP1903. See, *e.g.,* Straathof et al., Blood 105:4247-4254 (2005).

One can manufacture a clinical grade vector (such as a retroviral vector) and CAR-modified T-cell lines from MM patients and infuse them into patients with relapsed MM. One can evaluate the safety of the procedure and, if toxicity occurs, one can administer the dimerizer drug to activate the iC9 safety gene *in vivo.* One can also assess whether T-cell infusions provide disease control in patients with detectable disease.

It is an object of the disclosure to characterize the fate of the infused CAR-T cells by measuring their *in vivo* survival, and the subsequent effects of the dimerizing drug on these cells *in vitro* and - if clinically indicated - *in vivo.* One can also compare the accumulation of these cells in the BM and peripheral blood, the differential expression of the CAR by T cells in each environment, and their related ability to kill tumor cells. The cells and, when applicable, the dimerizing drug, may be provided clinically to an individual in need thereof.

Embodiments in this disclosure will elicit survivin-specific CTLs in MM patients by vaccination. In a complementary approach, the disclosure provides target neoplastic PC, making use of CAR technology in hematological malignancies.

### II. Hematological Malignancies

Hematological malignancies include cancer types that affect blood, bone marrow, and lymph nodes. They may derive from either of the two major blood cell lineages: myeloid or lymphoid cell lines. The myeloid cell line normally produces granulocytes, erythrocytes, thrombocytes, macrophages and mast cells; the lymphoid cell line produces B, T, NK and plasma cells. Lymphomas, lymphocytic leukemias, and myeloma are from the lymphoid line, while acute and chronic myelogenous leukemia, myelodysplastic syndromes and myeloproliferative diseases are myeloid in origin. In certain embodiments any of these haematological malignancies are treatable with methods and/or compositions of the disclosure.

Multiple myeloma (MM, which is also known as plasma cell myeloma or Kahler's disease) may be treated with methods and compositions of the present disclosure. MM is a cancer of plasma cells, which are a type of white blood cell normally responsible for producing antibodies. In MM, pluralities of abnormal plasma cells amass in the bone marrow, where they disrupt the production of normal blood cells. MM may be diagnosed in a variety of ways, such as with blood tests (serum protein electrophoresis or serum free kappa/lambda light chain assay, for example), bone marrow examination, urine protein electrophoresis, and X-rays of commonly involved bones. Although considered incurable, it may be treated with steroids, chemotherapy, proteasome inhibitors (e.g. bortezomib), immunomodulatory drugs (IMiDs) such as thalidomide or lenalidomide, radiation, and/or stem cell transplants. Certain symptoms include, for example, elevated calcium, renal failure, anemia, and/or bone lesions, for example. The treatment of the present disclosure is useful for Stage I, Stage II, or Stage III MM. Part of the workup for MM may include serum free light chain assay, skeletal survey, bone marrow biopsy, and/or quantitative measure of IgGs, for example.

In addition to embodiments of the disclosure, one with MM may be provided high-dose chemotherapy with autologous hematopoietic stem-cell transplantation, particularly if the individual is under the age of 65. Prior to stem-cell transplantation, these individuals may receive an initial course of induction chemotherapy, such as thalidomide, dexamethasone, bortezomib based regimens, lenalidomide, or combinations thereof. Autologous or allogeneic stem cell transplantation (ASCT) may be employed. Individuals over the age of 65 may be treated with melphalan, prednisone. bortezomib, melphalan, lenalidomide, or a combination thereof.

### III. CD138

CD138 (also known as syndecan 1) is a protein that in humans is encoded by the SDC1 gene. As an illustration, GenBank® Accession No. BC008765 provides an exemplary CD138 polynucleotide and GenBank® Accession No. AAH08765 provides an exemplary CD138 polypeptide, both of which are incorporated by reference herein in their entirety. Such sequences are useful to the skilled artisan to generate CD138-specific CAR molecules, for example.

### IV. Chimeric Antigen Receptors (CAR)

In some cases, cells are modified to express a CD138-specific CAR. Genetic engineering of human T lymphocytes to express tumor-directed chimeric antigen receptors (CAR) can produce antitumor effector cells that bypass tumor immune escape mechanisms that are due to abnormalities in protein-antigen processing and presentation. Moreover, these transgenic receptors can be directed to tumor-associated antigens that are not protein-derived. In certain embodiments of the disclosure there are CTLs that are modified to comprise at least a CAR. In specific aspects, a particular cell comprises expression of two or more CD138-specific recognition moieties, including two or more CD138-specific CARs.

The present disclosure includes an artificial T cell receptor referred to as a CAR (it also may be called chimeric T cell receptors or chimeric immunoreceptors). In embodiments of the disclosure it is specific for CD138. The CAR generally may include an ectodomain, transmembrane domain, and endodomain. It may be first generation, second generation, or third generation, in specific embodiments.

In particular cases, immune cells include a CAR that is chimeric, non-natural, engineered at least in part by the hand of man, and directed to CD138. In particular cases, the engineered CAR has one, two, three, four, or more components, and in some embodiments the one or more components facilitate targeting or binding of the T lymphocyte to the tumor antigen-comprising cancer cell. In specific embodiments, the CAR comprises an antibody for the tumor antigen, part or all of a cytoplasmic signaling domain, and/or part or all of one or more co-stimulatory molecules, for example endodomains of co-stimulatory molecules. In specific embodiments, the antibody is a single-chain variable fragment (scFv). In certain aspects the antibody is directed at target antigens on the cell surface of cancer cells that express CD138, for example. In certain embodiments, a cytoplasmic signaling domain, such as those derived from the T cell receptor ζ-chain, is employed as at least part of the chimeric receptor in order to produce stimulatory signals for T lymphocyte proliferation and effector function following engagement of the chimeric receptor with the target antigen. Examples would include, but are not limited to, endodomains from co-stimulatory molecules such as CD27, CD28, 4-1BB, and OX40 or the signaling components of cytokine receptors such as IL7 and IL15. In particular embodiments, co-stimulatory molecules are employed to enhance the activation, proliferation, and cytotoxicity of T cells produced by the CAR after antigen engagement. In specific embodiments, the co-stimulatory molecules are CD28, OX40, and 4-1BB and cytokine and the cytokine receptors are IL7 and IL15.

In general, an ectodomain of the CAR encompasses a signal peptide, antigen recognition domain, and a spacer that links the antigen recognition domain to the transmembrane domain. The antigen recognition domain generally will comprise a single chain variable fragment (scFv) specific for CD138. However, in cases wherein there are two or more CARs in the same cell, the second CAR may comprise an scFv specific for any one of Melanoma-associated antigen (MAGE), Preferentially expressed antigen of melanoma (PRAME), survivin, CD19, CD20, CD22, κ-light chain, CD30, CD33, CD123, CD38, ROR1,ErbB2,ErbB3/4, EGFr vIII, carcinoembryonic antigen, EGP2, EGP40, mesothelin, TAG72, PSMA, NKG2D ligands, B7-H6, IL-13 receptor a2, MUC1, MUC16, CA9, GD2, GD3, HMW-MAA, CD171, Lewis Y, G250/CAIX, HLA-AI MAGE A1, HLA-A2 NY-ESO-1, PSC1, folate receptor-a, CD44v6, CD44v7/8, 8H9, NCAM, VEGF receptors, 5T4, Fetal AchR, NKG2D ligands, or CD44v6, for example.

Examples of hinge regions for the ectodomain include the CH2CH3 region of immunoglobulin, the hinge region from IgG1, and portions of CD3. The transmembrane region may be of any kind, although in some cases it is CD28.

In general, the endodomain of the CAR of the disclosure is utilized for signal transmission in the cell after antigen recognition and cluster of the receptors. The most commonly used endodomain component is CD3-zeta that contains 3 ITAMs and that transmits an activation signal to the T cell after the antigen is bound. In some embodiments, additional co-stimulatory signaling is utilized, such as CD3-zeta in combination with CD28, 4-1BB, and/or OX40.

### V. Suicide Genes

In embodiments of the disclosure, a suicide gene is employed in particular expression vectors to permit the cell to kill itself through apoptosis at a desired point in time or location or physiological event, for example. The suicide gene may be present on the same expression vector as the CD138-CAR vector. Although suicide genes are known in the art and routinely used, in specific embodiments the suicide gene used in the disclosure is caspase 9, herpes simplex virus, thymidine kinase (HSV-tk), cytosine deaminase (CD) or cytochrome P450. In specific aspects the suicide gene is inducible and activated using a specific chemical inducer of dimerization (CID) (Ramos *et al.,* 2010).

### VI. Hypoxia-Responsive Regulatory Elements

Embodiments of the disclosure employ regulatory elements that allow environmental control of the activity of the therapeutic cells, including control of the expression of artificial antigen receptors in therapeutic cells. In specific embodiments, the expression of a CAR is regulated in an environmental or tissue-specific manner, such as in the presence of hypoxia or in hypoxic tissues. The one or more elements are utilized in the expression vector that encodes a CD138-specific CAR and is functionally related to the sequence that encodes the CD138-specific CAR. Functionally related can mean that the one or more elements are configured in a suitable manner to be able to modulate expression of the sequence that encodes the CD138-specific CAR polypeptide. Such elements by definition in a normoxic environment are not able to affect transcription of the CD138-specific CAR at all or to more than negligible, in some embodiments.

Exemplary HREs that may be employed include the sequence NCGTG, for example, and in particular embodiments the sequence is repeated in tandem, such as at least 6 times, although it may be 7,8, 9, 10, or more times.

### VII. Engager Molecules

In some embodiments of the disclosure, the cells of the disclosure that comprise a CD138-specific CAR also are modified to express an engager molecule. Such cells are capable of secreting a bi-specific T cell engager molecule, in particular embodiments. The bi-specificity in particular embodiments encompasses the presence of an activation domain and an antigen recognition domain. The antigen recognition domain binds to molecules that are present in or on target cells or that are secreted by target cells, and the activation domain binds to cell surface receptors present on T-cell, NK-cells, or NKT-cells that elicit processes that ultimately activate the recipient cell. The binding domain and antigen recognition domain may be of any kind, but in specific embodiments one scFv is specific for a cell surface molecule that mediates activation and the other scFv is specific for a particular tumor antigen of choice, including CD138 or another tumor antigen. The particular scFv for the tumor antigen may be tailored to recognize a corresponding cancer cell having the particular tumor antigen.

In particular aspects, the engager molecule comprises an activation domain that binds to an activation molecule on an immune cell surface (or an engineered immune cell surface), and an antigen recognition domain that binds to a target cell antigen, *e.g.,* an antigen expressed on the surface of a tumor cell or cancer cell.

The engager may be bipartite (*e.g.*, comprising an activation domain and antigen recognition domain that may optionally be joined by a linker), or may be tripartite or multipartite (*e.g.*, comprise one or more activation domains and/or antigen recognition domains, or other domains). In specific embodiments, the activation domain of the engager is or comprises an antibody or an antigen-binding fragment or portion thereof, *e.g.,* a single chain variable fragment (scFv). On other specific embodiments, the antigen recognition domain is or comprises an antibody or an antigen-binding fragment or portion thereof, *e.g.,* a monoclonal antibody or an scFv, or it may comprise ligands, peptides, soluble T-cell receptors, or combinations thereof. In certain embodiments, the activation domain and antigen recognition domain are joined by a linker, *e.g.,* a peptide linker.

The skilled artisan recognizes that immune cells have different activating receptors, and the engager will be tailored to the cell being activated. For example CD3 is an activating receptor on T-cells, whereas CD16, NKG2D, or NKp30 are activating receptors on NK cells, and CD3 or an invariant TCR are the activating receptors on NKT-cells. Engager molecules that activate T-cells may therefore have a different activation domain than engager molecules that activate NK cells. In specific embodiments, *e.g.,* wherein the immune cell is a T-cell, the activation molecule is one or more of CD3, *e.g.,* CD3γ, CD3δ or CD3ε; or CD27, CD28, CD40, CD134, CD137, and CD278. In other specific embodiments, *e.g.,* wherein the immune cell is a NK cell, the activation molecule is CD16, NKG2D, or NKp30, or wherein the immune cell is a NKT-cell, the activation molecule is CD3 or an invariant TCR.

In certain other embodiments, the engager additionally comprises one or more accessory domains, *e.g.,* one or more of a cytokine, a costimulatory domain, a domain that inhibits negative regulatory molecules of T-cell activation, or a combination thereof. In specific embodiments, the cytokine is IL-15, IL-2, and/or IL-7. In other specific embodiments, the costimulatory domain is CD27, CD80, CD86, CD134, or CD137. In other specific embodiments, the domain that inhibits negative regulatory molecules of T-cell activation is PD-1, PD-L1, CTLA4, or B7-H4.

In some instances, an scFV of the engager is specific for EphA2, CD19, 8H9, CAIX, CD20, CD30, CD33, CD44, CD70, CD123, CD138, EGFR, EGFRvIII, EGP2, EGP40, EPCAM, EphA2, ERBB2 (HER2), ERBB3, ERBB4, FAP, FAR, FBP, GD2, GD3, HLA-A1+MAGE1, IL11Rα, IL13Rα2, κ-light chain, KDR, Lambda, Lewis-Y, MCSP, Mesothelin, Muc1, NCAM, NKG2D ligands, TAG72, TEM1, TEM8, CEA, PSCA, and PSMA.

### VIII. Virus-Specificity

In embodiments of the disclosure, the CD138-CAR cells are CTLs whose native receptors are specific for virus latency proteins, such as those derived from EBV, HIV, HTLV-1, and so forth. These virus-specific CTLs can receive physiologic co-stimulation from professional antigen presenting cells processing latent viral antigens and kill tumor cells through their CAR. This dual specificity allows redirected-CTLs to receive physiologic costimulation from professional antigen presenting cells, whilst exploiting anti-tumor activity through the CAR.

One of skill in the art recognizes that it is routine to generate such types of cells.

### IX. Host Cells Comprising a CD138-Specific CAR

Embodiments of cells of the disclosure include those that are capable of expressing a CD138-specific CAR and include T-cells, NK-cells, and NKT-cells, for example. As used herein, the terms "cell," "cell line," and "cell culture" may be used interchangeably. All of these terms also include their progeny, which is any and all subsequent generations. It is understood that all progeny may not be identical due to deliberate or inadvertent mutations. In the context of expressing a heterologous nucleic acid sequence, a "host cell" can refer to a prokaryotic or eukaryotic cell, and it includes any transformable organism that is capable of replicating a vector and/or expressing a heterologous gene encoded by a vector. A host cell can, and has been, used as a recipient for vectors. A host cell may be "transfected" or "transformed," which refers to a process by which exogenous nucleic acid is transferred or introduced into the host cell. A transformed cell includes the primary subject cell and its progeny. As used herein, the terms "engineered" and "recombinant" cells or host cells are intended to refer to a cell into which an exogenous nucleic acid sequence, such as, for example, a vector, has been introduced. Therefore, recombinant cells are distinguishable from naturally occurring cells which do not contain a recombinantly introduced nucleic acid. In embodiments of the disclosure, a host cell is an immune cell, such as a T cell, including a cytotoxic T cell (also known as TC, Cytotoxic T Lymphocyte, CTL, T-Killer-cell, cytolytic T-cell, CD8+ T-cells or killer T cell); NK cells, NKT cells, and other immune cells that can elicit an effector function are also encompassed in the disclosure.

In certain embodiments, it is contemplated that RNAs or proteinaceous sequences may be co-expressed with other selected RNAs or proteinaceous sequences in the same host cell. Co-expression may be achieved by co-transfecting the host cell with two or more distinct recombinant vectors. Alternatively, a single recombinant vector may be constructed to include multiple distinct coding regions for RNAs, which could then be expressed in host cells transfected with the single vector.

Some vectors may employ control sequences that allow it to be replicated and/or expressed in both prokaryotic and eukaryotic cells. One of skill in the art would further understand the conditions under which to incubate all of the above described host cells to maintain them and to permit replication of a vector. Also understood and known are techniques and conditions that would allow large-scale production of vectors, as well as production of the nucleic acids encoded by vectors and their cognate polypeptides, proteins, or peptides.

The cells used in the disclosure are eukaryotic, including mammalian, although prokaryotic cells may be employed for manipulation in recombinant engineering of vectors or DNA to integrate into the vectors. The cells are particularly human, but can be associated with any animal of interest, particularly domesticated animals, such as equine, bovine, murine, ovine, canine, feline, *etc.* for use in their respective animal. Among these species, various types of cells can be involved, such as T cells, NK cells, NKT cells, *etc.*

The cells can be autologous cells, syngeneic cells, allogenic cells and even in some cases, xenogeneic cells, such as in relation to the individual that is receiving the cells. The cells may be modified by changing the major histocompatibility complex ("MHC") profile, by inactivating β₂-microglobulin to prevent the formation of functional Class I MHC molecules, inactivation of Class II molecules, providing for expression of one or more MHC molecules, enhancing or inactivating cytotoxic capabilities by enhancing or inhibiting the expression of genes associated with the cytotoxic activity, or the like.

In some instances specific clones or oligoclonal cells may be of interest, where the cells have a particular specificity, such as T cells and B cells having a specific antigen specificity or homing target site specificity.

Cells of the disclosure having a CD138-specific CAR may also express a second CAR, may also express an engager molecule, and/or may be viral-specific, and any recombinant expression construct may or may not be under the regulation of HREs.

Expression vectors that encode the CD138-specific CAR can be introduced into the cells as one or more DNA molecules or constructs, where there may be at least one marker that will allow for selection of host cells that contain the construct(s). The constructs can be prepared in conventional ways, where the genes and regulatory regions may be isolated, as appropriate, ligated, cloned in an appropriate cloning host, analyzed by restriction or sequencing, or other convenient means. Particularly, using PCR, individual fragments including all or portions of a functional unit may be isolated, where one or more mutations may be introduced using "primer repair", ligation, *in vitro* mutagenesis, *etc.,* as appropriate. The construct(s) once completed and demonstrated to have the appropriate sequences may then be introduced into the CTL by any convenient means. The constructs may be integrated and packaged into non-replicating, defective viral genomes like Adenovirus, Adeno-associated virus (AAV), or Herpes simplex virus (HSV) or others, including retroviral vectors, for infection or transduction into cells. The constructs may include viral sequences for transfection, if desired. Alternatively, the construct may be introduced by fusion, electroporation, biolistics, transfection, lipofection, or the like. The host cells may be grown and expanded in culture before introduction of the construct(s), followed by the appropriate treatment for introduction of the construct(s) and integration of the construct(s). The cells are then expanded and screened by virtue of a marker present in the construct. Various markers that may be used successfully include hprt, neomycin resistance, thymidine kinase, hygromycin resistance, *etc.*

In many situations one may wish to be able to kill the modified cells, where one wishes to terminate the treatment, the cells become neoplastic, in research where the absence of the cells after their presence is of interest, or other event. For this purpose one can provide for the expression of certain gene products in which one can kill the modified cells under controlled conditions. Suicide gene products, such as caspase 9, are examples of such products.

By way of illustration, cancer patients or patients susceptible to cancer or suspected of having cancer may be treated as follows. Cells modified as described herein may be administered to the patient and retained for extended periods of time. The individual may receive one or more administrations of the cells. Illustrative cells include hypoxia-responsive CD138-specific CAR T cells. The cell(s) would be modified at least to express at least hypoxia-responsive CD138-specific CAR and is provided to the individual in need thereof.

### X. Introduction of Constructs into Cells

The hypoxia-responsive CD138-specific CAR constructs, or any constructs described herein, can be introduced as one or more DNA molecules or constructs, where there may be at least one marker that will allow for selection of host cells that contain the construct(s). The constructs can be prepared in conventional ways, where the genes and regulatory regions may be isolated, as appropriate, ligated, cloned in an appropriate cloning host, analyzed by restriction or sequencing, or other convenient means. Particularly, using PCR, individual fragments including all or portions of a functional unit may be isolated, where one or more mutations may be introduced using "primer repair", ligation, *in vitro* mutagensis, *etc.* as appropriate. The construct(s) once completed and demonstrated to have the appropriate sequences may then be introduced into the host cell by any convenient means. The constructs may be integrated and packaged into non-replicating, defective viral genomes like Adenovirus, Adeno-associated virus (AAV), or Herpes simplex virus (HSV) or others, including retroviral vectors, for infection or transduction into cells. The constructs may include viral sequences for transfection, if desired. Alternatively, the construct may be introduced by fusion, electroporation, biolistics, transfection, lipofection, or the like. The host cells may be grown and expanded in culture before introduction of the construct(s), followed by the appropriate treatment for introduction of the construct(s) and integration of the construct(s). The cells are then expanded and screened by virtue of a marker present in the construct. Various markers that may be used successfully include hprt, neomycin resistance, thymidine kinase, hygromycin resistance, *etc.*

In some instances, one may have a target site for homologous recombination, where it is desired that a construct be integrated at a particular locus. For example,) can knock-out an endogenous gene and replace it (at the same locus or elsewhere) with the gene encoded for by the construct using materials and methods as are known in the art for homologous recombination. For homologous recombination, one may use either .OMEGA. or O-vectors. See, for example, Thomas and Capecchi, Cell (1987) 51, 503-512; Mansour, et al., Nature (1988) 336, 348-352; and Joyner, et al., Nature (1989) 338, 153-156.

Vectors containing useful elements such as bacterial or yeast origins of replication, selectable and/or amplifiable markers, promoter/enhancer elements for expression in prokaryotes or eukaryotes, *etc.* that may be used to prepare stocks of construct DNAs and for carrying out transfections are well known in the art, and many are commercially available.

### XI. Administration of Cells

The disclosure encompasses administration of cells to an individual in need thereof once the cells have been properly prepared, including engineering the cells to express the CD138-specific CAR and, at least in some cases, expanding the cells prior to administration to the individual.

The cells that have been modified with the DNA constructs are grown in culture under selective conditions and cells that are selected as having the construct may then be expanded and further analyzed, using, for example; the polymerase chain reaction for determining the presence of the construct in the host cells. Once the modified host cells have been identified or confirmed, they may then be used as planned, e.g. expanded in culture or introduced into a host organism.

Depending upon the nature of the cells, the cells may be introduced into a host organism, e.g. a mammal, in a wide variety of ways. The cells are introduced locally, such as in bone marrow, in specific embodiments, although in alternative embodiments the cells are given systemically and hone to the cancer or are modified to hone to the cancer. The number of cells which are employed will depend upon a number of circumstances, the purpose for the introduction, the lifetime of the cells, the protocol to be used, for example, the number of administrations, the ability of the cells to multiply, the stability of the recombinant construct, and the like. The cells may be applied as a dispersion, generally being injected at or near the site of interest. The cells may be in a physiologically-acceptable medium.

The DNA introduction need not result in integration in every case. In some situations, transient maintenance of the DNA introduced may be sufficient. In this way, one could have a short term effect, where cells could be introduced into the host and then turned on after a predetermined time, for example, after the cells have been able to home to a particular site.

The cells may be administered as desired. Depending upon the response desired, the manner of administration, the life of the cells, the number of cells present, various protocols may be employed. The number of administrations will depend upon the factors described above at least in part.

It should be appreciated that the system is subject to many variables, such as the cellular response to the ligand, the efficiency of expression and, as appropriate, the level of secretion, the activity of the expression product, the particular need of the patient, which may vary with time and circumstances, the rate of loss of the cellular activity as a result of loss of cells or expression activity of individual cells, and the like. Therefore, it is expected that for each individual patient, even if there were universal cells which could be administered to the population at large, each patient would be monitored for the proper dosage for the individual, and such practices of monitoring a patient are routine in the art.

CAR-modifed T-cells are administered *via* intravenous infusion. Doses can range from 1x10⁷/m² to 2x10⁸/m², for example.

In particular cases, a plurality of CD138 CAR-expressing immune cells are delivered to an individual with cancer that expresses the CD138 antigen. In specific embodiments, a single administration is required. In other embodiments, a plurality of administration of cells is required. For example, following a first administration of the engineered immune cells, there may be examination of the individual for the presence or absence of the cancer or for a reduction in the number and/or size of tumors, for example. In the event that the cancer shows a need for further treatment, such as upon tumor growth after the first administration, an additional one or more deliveries of the same CD138 CAR-expressing cells (or, optionally, another type of cancer therapy, including another type of immunotherapy, and/or chemotherapy, surgery and/or radiation) is given to the individual. In some cases, a reduction of tumor size in an individual indicates that the CD138 CAR-expressing immunotherapy is effective, so further administrations are provided to the individual.

### XI. Polynucleotides Encoding CD138-Specific CARs

The present disclosure also encompasses a composition comprising a nucleic acid sequence encoding a CD138-specific CAR as defined herein and cells harboring the nucleic acid sequence. The nucleic acid molecule is a recombinant nucleic acid molecule, in particular aspects and may be synthetic. It may comprise DNA, RNA as well as PNA (peptide nucleic acid) and it may be a hybrid thereof.

Furthermore, it is envisaged for further purposes that nucleic acid molecules may contain, for example, thioester bonds and/or nucleotide analogues. The modifications may be useful for the stabilization of the nucleic acid molecule against endo- and/or exonucleases in the cell. The nucleic acid molecules may be transcribed by an appropriate vector comprising a chimeric gene that allows for the transcription of said nucleic acid molecule in the cell. In this respect, it is also to be understood that such polynucleotides can be used for "gene targeting" or "gene therapeutic" approaches. In another embodiment the nucleic acid molecules are labeled. Methods for the detection of nucleic acids are well known in the art, e.g., Southern and Northern blotting, PCR or primer extension. This embodiment may be useful for screening methods for verifying successful introduction of the nucleic acid molecules described above during gene therapy approaches.

The nucleic acid molecule(s) may be a recombinantly produced chimeric nucleic acid molecule comprising any of the aforementioned nucleic acid molecules either alone or in combination. In specific aspects, the nucleic acid molecule is part of a vector.

The present disclosure therefore also relates to a composition comprising a vector comprising the nucleic acid molecule described in the present disclosure.

Many suitable vectors are known to those skilled in molecular biology, the choice of which would depend on the function desired and include plasmids, cosmids, viruses, bacteriophages and other vectors used conventionally in genetic engineering. Methods that are well known to those skilled in the art can be used to construct various plasmids and vectors; see, for example, the techniques described in Sambrook et al. (1989) and Ausubel, Current Protocols in Molecular Biology, Green Publishing Associates and Wiley Interscience, N.Y. (1989), (1994). Alternatively, the polynucleotides and vectors of the disclosure can be reconstituted into liposomes for delivery to target cells. A cloning vector may be used to isolate individual sequences of DNA. Relevant sequences can be transferred into expression vectors where expression of a particular polypeptide is required. Typical cloning vectors include pBluescript SK, pGEM, pUC9, pBR322 and pGBT9. Typical expression vectors include pTRE, pCAL-n-EK, pESP-1, pOP13CAT.

In specific embodiments, there is a vector that comprises a nucleic acid sequence that is a regulatory sequence operably linked to the nucleic acid sequence encoding a CD138-specific CAR defined herein. Such regulatory sequences (control elements) are known to the artisan and may include a promoter, a splice cassette, translation initiation codon, translation and insertion site for introducing an insert into the vector. In specific embodiments, the nucleic acid molecule is operatively linked to said expression control sequences allowing expression in eukaryotic or prokaryotic cells.

It is envisaged that a vector is an expression vector comprising the nucleic acid molecule encoding a CD138-specific CAR as defined herein. In specific aspects, the vector is a viral vector, such as a lentiviral vector. Lentiviral vectors are commercially available, including from Clontech (Mountain View, CA) or GeneCopoeia (Rockville, MD), for example.

The term "regulatory sequence" refers to DNA sequences that are necessary to effect the expression of coding sequences to which they are ligated. The nature of such control sequences differs depending upon the host organism. In prokaryotes, control sequences generally include promoters, ribosomal binding sites, and terminators. In eukaryotes generally control sequences include promoters, terminators and, in some instances, enhancers, transactivators or transcription factors. The term "control sequence" is intended to include, at a minimum, all components the presence of which are necessary for expression, and may also include additional advantageous components.

The term "operably linked" refers to a juxtaposition wherein the components so described are in a relationship permitting them to function in their intended manner. A control sequence "operably linked" to a coding sequence is ligated in such a way that expression of the coding sequence is achieved under conditions compatible with the control sequences. In case the control sequence is a promoter, it is obvious for a skilled person that double-stranded nucleic acid is preferably used.

Thus, the recited vector is an expression vector, in certain embodiments. An "expression vector" is a construct that can be used to transform a selected host and provides for expression of a coding sequence in the selected host. Expression vectors can for instance be cloning vectors, binary vectors or integrating vectors. Expression comprises transcription of the nucleic acid molecule preferably into a translatable mRNA. Regulatory elements ensuring expression in prokaryotes and/or eukaryotic cells are well known to those skilled in the art. In the case of eukaryotic cells they comprise normally promoters ensuring initiation of transcription and optionally poly-A signals ensuring termination of transcription and stabilization of the transcript. Possible regulatory elements permitting expression in prokaryotic host cells comprise, *e.g.,* the P_{L}, lac, trp or tac promoter in *E. coli,* and examples of regulatory elements permitting expression in eukaryotic host cells are the AOX1 or GAL1 promoter in yeast or the CMV-, SV40-, RSV-promoter (Rous sarcoma virus), CMV-enhancer, SV40-enhancer or a globin intron in mammalian and other animal cells.

Beside elements that are responsible for the initiation of transcription such regulatory elements may also comprise transcription termination signals, such as the SV40-poly-A site or the tk-poly-A site, downstream of the polynucleotide. Furthermore, depending on the expression system used leader sequences capable of directing the polypeptide to a cellular compartment or secreting it into the medium may be added to the coding sequence of the recited nucleic acid sequence and are well known in the art. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including an N-terminal identification peptide imparting desired characteristics, e.g., stabilization or simplified purification of expressed recombinant product; see supra. In this context, suitable expression vectors are known in the art such as Okayama-Berg cDNA expression vector pcDV1 (Pharmacia), pEF-Neo, pCDM8, pRc/CMV, pcDNA1, pcDNA3 (Invitrogen), pEF-DHFR and pEF-ADA, (Raum et al. Cancer Immunol Immunother (2001) 50(3), 141-150) or pSPORT1 (GIBCO BRL).

In some embodiments, the expression control sequences are eukaryotic promoter systems in vectors capable of transforming of transfecting eukaryotic host cells, but control sequences for prokaryotic hosts may also be used. Once the vector has been incorporated into the appropriate host, the host is maintained under conditions suitable for high level expression of the nucleotide sequences, and as desired, the collection and purification of the polypeptide of the disclosure may follow. In particular embodiments, one or more encodable sequences are regulated by expression control sequences that are responsive to hypoxic environments.

Additional regulatory elements may include transcriptional as well as translational enhancers. Advantageously, the above-described vectors of the disclosure comprises a selectable and/or scorable marker. Selectable marker genes useful for the selection of transformed cells are well known to those skilled in the art and comprise, for example, antimetabolite resistance as the basis of selection for dhfr, which confers resistance to methotrexate (Reiss, Plant Physiol. (Life-Sci. Adv.) 13 (1994), 143-149); npt, which confers resistance to the aminoglycosides neomycin, kanamycin and paromycin (Herrera-Estrella, EMBO J. 2 (1983), 987-995) and hygro, which confers resistance to hygromycin (Marsh, Gene 32 (1984), 481-485). Additional selectable genes have been described, namely trpB, which allows cells to utilize indole in place of tryptophan; hisD, which allows cells to utilize histinol in place of histidine (Hartman, Proc. Natl. Acad. Sci. USA 85 (1988), 8047); mannose-6-phosphate isomerase which allows cells to utilize mannose (WO 94/20627) and ODC (ornithine decarboxylase) which confers resistance to the ornithine decarboxylase inhibitor, 2-(difluoromethyl)-DL-ornithine, DFMO (McConlogue, 1987, In: Current Communications in Molecular Biology, Cold Spring Harbor Laboratory ed.) or deaminase from Aspergillus terreus which confers resistance to Blasticidin S (Tamura, Biosci. Biotechnol. Biochem. 59 (1995), 2336-2338).

Useful scorable markers are also known to those skilled in the art and are commercially available. Advantageously, said marker is a gene encoding luciferase (Giacomin, P1. Sci. 116 (1996), 59-72; Scikantha, J. Bact. 178 (1996), 121), green fluorescent protein (Gerdes, FEBS Lett. 389 (1996), 44-47) or β-glucuronidase (Jefferson, EMBO J. 6 (1987), 3901-3907). This embodiment is particularly useful for simple and rapid screening of cells, tissues and organisms containing a recited vector.

As described above, the recited nucleic acid molecule can be used in a cell, alone, or as part of a vector to express the encoded polypeptide in cells. The nucleic acid molecules or vectors containing the DNA sequence(s) encoding any one of the CD138-specific CAR constructs is introduced into the cells that in turn produce the polypeptide of interest. The recited nucleic acid molecules and vectors may be designed for direct introduction or for introduction *via* liposomes, or viral vectors (*e.g.,* adenoviral, retroviral) into a cell. In certain embodiments, the cells are T-cells, CAR T-cells, NK cells, NKT-cells, MSCs, neuronal stem cells, or hematopoietic stem cells, for example.

In accordance with the above, the present disclosure relates to methods to derive vectors, particularly plasmids, cosmids, viruses and bacteriophages used conventionally in genetic engineering that comprise a nucleic acid molecule encoding the polypeptide sequence of a CD138-specific CAR defined herein. Preferably, said vector is an expression vector and/or a gene transfer or targeting vector. Expression vectors derived from viruses such as retroviruses, vaccinia virus, adeno-associated virus, herpes viruses, or bovine papilloma virus, may be used for delivery of the recited polynucleotides or vector into targeted cell populations. Methods which are well known to those skilled in the art can be used to construct recombinant vectors; see, for example, the techniques described in Sambrook *et al.* (loc cit.), Ausubel (1989, loc cit.) or other standard text books. Alternatively, the recited nucleic acid molecules and vectors can be reconstituted into liposomes for delivery to target cells. The vectors containing the nucleic acid molecules of the disclosure can be transferred into the host cell by well-known methods, which vary depending on the type of cellular host. For example, calcium chloride transfection is commonly utilized for prokaryotic cells, whereas calcium phosphate treatment or electroporation may be used for other cellular hosts; see Sambrook, supra.

### XII. Vectors Generally

Vectors of the disclosure may be used for recombinant engineering to produce and at least in some cases express, a CD138-specific recognition moiety, including a CD138-specific CAR or a CD138-specific engager molecule.

The term "vector" is used to refer to a carrier nucleic acid molecule into which a nucleic acid sequence can be inserted for introduction into a cell where it can be replicated. A nucleic acid sequence can be "exogenous," which means that it is foreign to the cell into which the vector is being introduced or that the sequence is homologous to a sequence in the cell but in a position within the host cell nucleic acid in which the sequence is ordinarily not found. Vectors include plasmids, cosmids, viruses (bacteriophage, animal viruses, and plant viruses), and artificial chromosomes (*e.g.*, YACs). One of skill in the art would be well equipped to construct a vector through standard recombinant techniques (see, for example, Maniatis *et al.,* 1988 and Ausubel *et al.,* 1994, both incorporated herein by reference).

The term "expression vector" refers to any type of genetic construct comprising a nucleic acid coding for a RNA capable of being transcribed. In some cases, RNA molecules are then translated into a protein, polypeptide, or peptide. In other cases, these sequences are not translated, for example, in the production of antisense molecules or ribozymes. Expression vectors can contain a variety of "control sequences," which refer to nucleic acid sequences necessary for the transcription and possibly translation of an operably linked coding sequence in a particular host cell. In addition to control sequences that govern transcription and translation, vectors and expression vectors may contain nucleic acid sequences that serve other functions as well and are described *infra.*

Regulatory sequences employed in the disclosure include one or more hypoxia responsive elements that are functionally linked to the expression construct of which the expression is regulated. The following describes other regulatory elements that may be employed.

A "promoter" is a control sequence that is a region of a nucleic acid sequence at which initiation and rate of transcription are controlled. It may contain genetic elements at which regulatory proteins and molecules may bind, such as RNA polymerase and other transcription factors, to initiate the specific transcription a nucleic acid sequence. The phrases "operatively positioned," "operatively linked," "under control," and "under transcriptional control" mean that a promoter is in a correct functional location and/or orientation in relation to a nucleic acid sequence to control transcriptional initiation and/or expression of that sequence.

A promoter generally comprises a sequence that functions to position the start site for RNA synthesis. The best known example of this is the TATA box, but in some promoters lacking a TATA box, such as, for example, the promoter for the mammalian terminal deoxynucleotidyl transferase gene and the promoter for the SV40 late genes, a discrete element overlying the start site itself helps to fix the place of initiation. Additional promoter elements regulate the frequency of transcriptional initiation. Typically, these are located in the region 30-110 bp upstream of the start site, although a number of promoters have been shown to contain functional elements downstream of the start site as well. To bring a coding sequence "under the control of" a promoter, one positions the 5' end of the transcription initiation site of the transcriptional reading frame "downstream" of (*i.e.,* 3' of) the chosen promoter. The "upstream" promoter stimulates transcription of the DNA and promotes expression of the encoded RNA.

The spacing between promoter elements frequently is flexible, so that promoter function is preserved when elements are inverted or moved relative to one another. In the tk promoter, the spacing between promoter elements can be increased to 50 bp apart before activity begins to decline. Depending on the promoter, it appears that individual elements can function either cooperatively or independently to activate transcription. A promoter may or may not be used in conjunction with an "enhancer," which refers to a *cis*-acting regulatory sequence involved in the transcriptional activation of a nucleic acid sequence.

A promoter may be one naturally associated with a nucleic acid sequence, as may be obtained by isolating the 5' non-coding sequences located upstream of the coding segment and/or exon. Such a promoter can be referred to as "endogenous." Similarly, an enhancer may be one naturally associated with a nucleic acid sequence, located either downstream or upstream of that sequence. Alternatively, certain advantages will be gained by positioning the coding nucleic acid segment under the control of a recombinant or heterologous promoter, which refers to a promoter that is not normally associated with a nucleic acid sequence in its natural environment. A recombinant or heterologous enhancer refers also to an enhancer not normally associated with a nucleic acid sequence in its natural environment. Such promoters or enhancers may include promoters or enhancers of other genes, and promoters or enhancers isolated from any other virus, or prokaryotic or eukaryotic cell, and promoters or enhancers not "naturally occurring," *i.e*., containing different elements of different transcriptional regulatory regions, and/or mutations that alter expression. For example, promoters that are most commonly used in recombinant DNA construction include the β-lactamase (penicillinase), lactose and tryptophan (trp) promoter systems. In addition to producing nucleic acid sequences of promoters and enhancers synthetically, sequences may be produced using recombinant cloning and/or nucleic acid amplification technology, including PCR™, in connection with the compositions disclosed herein (see U.S. Patent Nos. 4,683,202 and 5,928,906, each incorporated herein by reference). Furthermore, it is contemplated the control sequences that direct transcription and/or expression of sequences within non-nuclear organelles such as mitochondria, chloroplasts, and the like, can be employed as well.

Naturally, it will be important to employ a promoter and/or enhancer that effectively directs the expression of the DNA segment in the organelle, cell type, tissue, organ, or organism chosen for expression. Those of skill in the art of molecular biology generally know the use of promoters, enhancers, and cell type combinations for protein expression, (see, for example Sambrook *et al.* 1989, incorporated herein by reference). The promoters employed may be constitutive, tissue-specific, inducible, and/or useful under the appropriate conditions to direct high level expression of the introduced DNA segment, such as is advantageous in the large-scale production of recombinant proteins and/or peptides. The promoter may be heterologous or endogenous.

Additionally any promoter/enhancer combination could also be used to drive expression. Use of a T3, T7 or SP6 cytoplasmic expression system is another possible embodiment. Eukaryotic cells can support cytoplasmic transcription from certain bacterial promoters if the appropriate bacterial polymerase is provided, either as part of the delivery complex or as an additional genetic expression construct.

The identity of tissue-specific promoters or elements, as well as assays to characterize their activity, is well known to those of skill in the art. In specific embodiments, environment-specific promoters or elements are utilized, such as hypoxic-specific regulatory elements. Tissue-specific, lineage-specific, and/or activation- specific promoters may be employed, and examples include activated T-cell elements, NFAT (lineage-restricted activation), Early growth response gene (activation), liver X receptor response elements (activation), Hypoxia Response elements (environmental), *etc.*

A specific initiation signal also may be required for efficient translation of coding sequences. These signals include the ATG initiation codon or adjacent sequences. Exogenous translational control signals, including the ATG initiation codon, may need to be provided. One of ordinary skill in the art would readily be capable of determining this and providing the necessary signals.

In certain embodiments of the disclosure, the use of internal ribosome entry sites (IRES) elements are used to create multigene, or polycistronic, messages, and these may be used in the disclosure.

Vectors can include a multiple cloning site (MCS), which is a nucleic acid region that contains multiple restriction enzyme sites, any of which can be used in conjunction with standard recombinant technology to digest the vector. "Restriction enzyme digestion" refers to catalytic cleavage of a nucleic acid molecule with an enzyme that functions only at specific locations in a nucleic acid molecule. Many of these restriction enzymes are commercially available. Use of such enzymes is widely understood by those of skill in the art. Frequently, a vector is linearized or fragmented using a restriction enzyme that cuts within the MCS to enable exogenous sequences to be ligated to the vector. "Ligation" refers to the process of forming phosphodiester bonds between two nucleic acid fragments, which may or may not be contiguous with each other. Techniques involving restriction enzymes and ligation reactions are well known to those of skill in the art of recombinant technology.

Splicing sites, termination signals, origins of replication, and selectable markers may also be employed.

In certain embodiments, a plasmid vector is contemplated for use to transform a host cell. In general, plasmid vectors containing replicon and control sequences which are derived from species compatible with the host cell are used in connection with these hosts. The vector ordinarily carries a replication site, as well as marking sequences which are capable of providing phenotypic selection in transformed cells. In a non-limiting example, E. coli is often transformed using derivatives of pBR322, a plasmid derived from an E. coli species. pBR322 contains genes for ampicillin and tetracycline resistance and thus provides easy means for identifying transformed cells. The pBR plasmid, or other microbial plasmid or phage must also contain, or be modified to contain, for example, promoters which can be used by the microbial organism for expression of its own proteins.

In addition, phage vectors containing replicon and control sequences that are compatible with the host microorganism can be used as transforming vectors in connection with these hosts. For example, the phage lambda GEM™-11 may be utilized in making a recombinant phage vector which can be used to transform host cells, such as, for example, E. coli LE392.

Further useful plasmid vectors include pIN vectors (Inouye *et al.,* 1985); and pGEX vectors, for use in generating glutathione S-transferase (GST) soluble fusion proteins for later purification and separation or cleavage. Other suitable fusion proteins are those with β-galactosidase, ubiquitin, and the like.

Bacterial host cells, for example, E. coli, comprising the expression vector, are grown in any of a number of suitable media, for example, LB. The expression of the recombinant protein in certain vectors may be induced, as would be understood by those of skill in the art, by contacting a host cell with an agent specific for certain promoters, e.g., by adding IPTG to the media or by switching incubation to a higher temperature. After culturing the bacteria for a further period, generally of between 2 and 24 h, the cells are collected by centrifugation and washed to remove residual media.

### A. Viral Vectors

The ability of certain viruses to infect cells or enter cells *via* receptor-mediated endocytosis, and to integrate into host cell genome and express viral genes stably and efficiently have made them attractive candidates for the transfer of foreign nucleic acids into cells (*e.g.*, mammalian cells). Components of the present disclosure may be a viral vector that encodes heparanase. Non-limiting examples of virus vectors that may be used to deliver a nucleic acid of the present disclosure are described below.

### 1. Adenoviral Vectors

A particular method for delivery of the nucleic acid involves the use of an adenovirus expression vector. Although adenovirus vectors are known to have a low capacity for integration into genomic DNA, this feature is counterbalanced by the high efficiency of gene transfer afforded by these vectors. "Adenovirus expression vector" is meant to include those constructs containing adenovirus sequences sufficient to (a) support packaging of the construct and (b) to ultimately express a tissue or cell-specific construct that has been cloned therein. Knowledge of the genetic organization or adenovirus, a 36 kb, linear, double-stranded DNA virus, allows substitution of large pieces of adenoviral DNA with foreign sequences up to 7 kb (Grunhaus and Horwitz, 1992).

### 2. AAV Vectors

The nucleic acid may be introduced into the cell using adenovirus assisted transfection. Increased transfection efficiencies have been reported in cell systems using adenovirus coupled systems (Kelleher and Vos, 1994; Cotten *et al.,* 1992; Curiel, 1994). Adeno-associated virus (AAV) is an attractive vector system for use in the cells of the present disclosure as it has a high frequency of integration and it can infect nondividing cells, thus making it useful for delivery of genes into mammalian cells, for example, in tissue culture (Muzyczka, 1992) or *in vivo.* AAV has a broad host range for infectivity (Tratschin *et al.,* 1984; Laughlin *et al.,* 1986; Lebkowski *et al.,* 1988; McLaughlin *et al.,* 1988). Details concerning the generation and use of rAAV vectors are described in U.S. Patent Nos. 5,139,941 and 4,797,368, each incorporated herein by reference.

### 3. Retroviral Vectors

Retroviruses are useful as delivery vectors because of their ability to integrate their genes into the host genome, transferring a large amount of foreign genetic material, infecting a broad spectrum of species and cell types and of being packaged in special cell-lines (Miller, 1992).

In order to construct a heparanase retroviral vector, a nucleic acid (*e.g.,* one encoding part or all of heparanase) is inserted into the viral genome in the place of certain viral sequences to produce a virus that is replication-defective. In order to produce virions, a packaging cell line containing the *gag, pol,* and *env* genes but without the LTR and packaging components is constructed (Mann *et al.,* 1983). When a recombinant plasmid containing a cDNA, together with the retroviral LTR and packaging sequences is introduced into a special cell line (*e.g.,* by calcium phosphate precipitation for example), the packaging sequence allows the RNA transcript of the recombinant plasmid to be packaged into viral particles, which are then secreted into the culture media (Nicolas and Rubenstein, 1988; Temin, 1986; Mann *et al.,* 1983). The media containing the recombinant retroviruses is then collected, optionally concentrated, and used for gene transfer. Retroviral vectors are able to infect a broad variety of cell types. However, integration and stable expression require the division of host cells (Paskind *et al.,* 1975).

Lentiviruses are complex retroviruses, which, in addition to the common retroviral genes *gag, pol,* and *env,* contain other genes with regulatory or structural function. Lentiviral vectors are well known in the art (see, for example, Naldini *et al.,* 1996; Zufferey *et al.,* 1997; Blomer *et al.,* 1997; U.S. Pat. Nos. 6,013,516 and 5,994,136). Some examples of lentivirus include the Human Immunodeficiency Viruses: HIV-1, HIV-2 and the Simian Immunodeficiency Virus: SIV. Lentiviral vectors have been generated by multiply attenuating the HIV virulence genes, for example, the genes *env, vif, vpr, vpu* and *nef* are deleted making the vector biologically safe.

Recombinant lentiviral vectors are capable of infecting non-dividing cells and can be used for both *in vivo* and *ex vivo* gene transfer and expression of nucleic acid sequences. For example, recombinant lentivirus capable of infecting a non-dividing cell wherein a suitable host cell is transfected with two or more vectors carrying the packaging functions, namely gag, pol and env, as well as rev and tat is described in U.S. Pat. No. 5,994,136, incorporated herein by reference. One may target the recombinant virus by linkage of the envelope protein with an antibody or a particular ligand for targeting to a receptor of a particular cell-type. By inserting a sequence (including a regulatory region) of interest into the viral vector, along with another gene which encodes the ligand for a receptor on a specific target cell, for example, the vector is now target-specific.

### 4. Other Viral Vectors

Other viral vectors may be employed as vaccine constructs in the present disclosure. Vectors derived from viruses such as vaccinia virus (Ridgeway, 1988; Baichwal and Sugden, 1986; Coupar *et al.,* 1988), sindbis virus, cytomegalovirus and herpes simplex virus may be employed. They offer several attractive features for various mammalian cells (Friedmann, 1989; Ridgeway, 1988; Baichwal and Sugden, 1986; Coupar *et al.,* 1988; Horwich *et al.,* 1990).

### 5. Delivery Using Modified Vectors

A nucleic acid to be delivered may be housed within an infective virus that has been engineered to express a specific binding ligand. The virus particle will thus bind specifically to the cognate receptors of the target cell and deliver the contents to the cell. A novel approach designed to allow specific targeting of retrovirus vectors was developed based on the chemical modification of a retrovirus by the chemical addition of lactose residues to the viral envelope. This modification can permit the specific infection of hepatocytes *via* sialoglycoprotein receptors.

Another approach to targeting of recombinant retroviruses was designed in which biotinylated antibodies against a retroviral envelope protein and against a specific cell receptor were used. The antibodies were coupled *via* the biotin components by using streptavidin (Roux *et al.,* 1989). Using antibodies against major histocompatibility complex class I and class II antigens, they demonstrated the infection of a variety of human cells that bore those surface antigens with an ecotropic virus *in vitro* (Roux *et al.,* 1989).

### B. Vector Delivery and Cell Transformation

Suitable methods for nucleic acid delivery for transfection or transformation of cells are known to one of ordinary skill in the art. Such methods include, but are not limited to, direct delivery of DNA such as by *ex vivo* transfection, by injection, and so forth. Through the application of techniques known in the art, cells may be stably or transiently transformed.

### C. Ex Vivo Transformation

Methods for tranfecting eukaryotic cells and tissues removed from an organism in an *ex vivo* setting are known to those of skill in the art. Thus, it is contemplated that cells or tissues may be removed and transfected ex vivo using heparanase or other nucleic acids of the present disclosure. In particular aspects, the transplanted cells or tissues may be placed into an organism. In preferred facets, a nucleic acid is expressed in the transplanted cells.

### XIII. Combination Therapy

In certain embodiments of the disclosure, methods of the present disclosure for clinical aspect, e.g., administration to an individual having a CD138-expresssing cancer immune cells, *e.g.,* T cells, expressing a CD138-specific CAR, are combined with other agents effective in the treatment of hyperproliferative disease, such as anti-cancer agents. An "anti-cancer" agent is capable of negatively affecting cancer in a subject, for example, by killing cancer cells, inducing apoptosis in cancer cells, reducing the growth rate of cancer cells, reducing the incidence or number of metastases, reducing tumor size, inhibiting tumor growth, reducing the blood supply to a tumor or cancer cells, promoting an immune response against cancer cells or a tumor, preventing or inhibiting the progression of cancer, or increasing the lifespan of a subject with cancer. More generally, these other compositions would be provided in a combined amount effective to kill or inhibit proliferation of the cell. This process may involve contacting the cancer cells with the expression construct and the agent(s) or multiple factor(s) at the same time. This may be achieved by contacting the cell with a single composition or pharmacological formulation that includes both agents, or by contacting the cell with two distinct compositions or formulations, at the same time, wherein one composition includes the expression construct and the other includes the second agent(s).

In embodiments of the disclosure, one or more of the following are provided to an individual with MM in addition to the therapeutic cells of the disclosure: steroids, chemotherapy, proteasome inhibitors (*e.g.* bortezomib), immunomodulatory drugs (IMiDs) such as thalidomide or lenalidomide, radiation, and/or stem cell transplants.

Tumor cell resistance to chemotherapy and radiotherapy agents represents a major problem in clinical oncology. One goal of current cancer research is to find ways to improve the efficacy of chemo- and radiotherapy by combining it with gene therapy. For example, the herpes simplex-thymidine kinase (HS-tK) gene, when delivered to brain tumors by a retroviral vector system, successfully induced susceptibility to the antiviral agent ganciclovir (Culver, *et al.,* 1992). In the context of the present disclosure, it is contemplated that cell therapy could be used similarly in conjunction with chemotherapeutic, radiotherapeutic, or immunotherapeutic intervention, in addition to other pro-apoptotic or cell cycle regulating agents.

Alternatively, the present inventive therapy may precede or follow the other agent treatment by intervals ranging from minutes to weeks. In embodiments where the other agent and present disclosure are applied separately to the individual, one would generally ensure that a significant period of time did not expire between the time of each delivery, such that the agent and inventive therapy would still be able to exert an advantageously combined effect on the cell. In such instances, it is contemplated that one may contact the cell with both modalities within about 12-24 h of each other and, more preferably, within about 6-12 h of each other. In some situations, it may be desirable to extend the time period for treatment significantly, however, where several d (2, 3, 4, 5, 6 or 7) to several wk (1, 2, 3, 4, 5, 6, 7 or 8) lapse between the respective administrations.

Various combinations may be employed, present disclosure is "A" and the secondary agent, such as radio- or chemotherapy, is "B":

| | | | | | |
|---|---|---|---|---|---|
| A/B/A | B/A/B B/B/A | A/A/B | A/B/B B/A/A | A/B/B/B | B/A/B/B |
| B/B/B/A | B/B/A/B | A/A/B/B | A/B/A/B | A/B/B/A | B/B/A/A |
| B/A/B/A | B/A/A/B | A/A/A/B | B/A/A/A | A/B/A/A | A/A/B/A |

It is expected that the treatment cycles would be repeated as necessary. It also is contemplated that various standard therapies, as well as surgical intervention, may be applied in combination with the inventive cell therapy.

### A. Chemotherapy

Cancer therapies also include a variety of combination therapies with both chemical and radiation based treatments. Combination chemotherapies include, for example, acivicin; aclarubicin; acodazole hydrochloride; acronine; adozelesin; aldesleukin; altretamine; ambomycin; ametantrone acetate; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; celecoxib (COX-2 inhibitor); chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflomithine hydrochloride; elsamitrucin; enloplatin; enpromate; epipropidine; epirubicin hydrochloride; erbulozole; esorubicin hydrochloride; estrarnustine; estramustine phosphate sodium; etanidazole; etoposide; etoposide phosphate; etoprine; fadrozole hydrochloride; fazarabine; fenretinide; floxuridine; fludarabine phosphate; fluorouracil; fluorocitabine; fosquidone; fostriecin sodium; gemcitabine; gemcitabine hydrochloride; hydroxyurea; idarubicin hydrochloride; ifosfamide; ilmofosine; iproplatin; irinotecan; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; taxotere; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride; 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidenmin B; deslorelin; dexamethasone; dexifosfamide; dexrazoxane; dexverapamil; diaziquone: didemnin B; didox; diethylnorspermine; dihydro-5-azacytidine; dihydrotaxol, 9-; dioxamycin; diphenyl spiromustine; docetaxel; docosanol; dolasetron; doxifluridine; doxorubicin; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imatinib (e.g., GLEEVEC®), imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mitoguazone; mitolactol; mitomycin analogues; mitonafide; mitotoxin fibroblast growth factor-saporin; mitoxantrone; mofarotene; molgramostim; Erbitux, human chorionic gonadotrophin; monophosphoryl lipid A+myobacterium cell wall sk; mopidamol; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin: neridronic acid; nilutamide; nisamycin; nitric oxide modulators; nitroxide antioxidant; nitrullyn; oblimersen (GENASENSE®); O⁶-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; sizofuran; sobuzoxane; sodium borocaptate; sodium phenylacetate; solverol; somatomedin binding protein; sonermin; sparfosic acid; spicamycin D; spiromustine; splenopentin; spongistatin 1; squalamine; stipiamide; stromelysin inhibitors; sulfinosine; superactive vasoactive intestinal peptide antagonist; suradista; suramin; swainsonine; tallimustine; tamoxifen methiodide; tauromustine; tazarotene; tecogalan sodium; tegafur; tellurapyrylium; telomerase inhibitors; temoporfin; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; velaresol; veramine; verdins; verteporfin; vinorelbine; vinxaltine; vitaxin; vorozole; zanoterone; zeniplatin; zilascorb; and zinostatin stimalamer, or any analog or derivative variant of the foregoing.

### B Radiotherapy

Other factors that cause DNA damage and have been used extensively include what are commonly known as γ-rays, X-rays, and/or the directed delivery of radioisotopes to tumor cells. Other forms of DNA damaging factors are also contemplated such as microwaves and UV-irradiation. It is most likely that all of these factors effect a broad range of damage on DNA, on the precursors of DNA, on the replication and repair of DNA, and on the assembly and maintenance of chromosomes. Dosage ranges for X-rays range from daily doses of 50 to 200 roentgens for prolonged periods of time (3 to 4 wk), to single doses of 2000 to 6000 roentgens. Dosage ranges for radioisotopes vary widely, and depend on the half-life of the isotope, the strength and type of radiation emitted, and the uptake by the neoplastic cells.

The terms "contacted" and "exposed," when applied to a cell, are used herein to describe the process by which a therapeutic construct and a chemotherapeutic or radiotherapeutic agent are delivered to a target cell or are placed in direct juxtaposition with the target cell. To achieve cell killing or stasis, both agents are delivered to a cell in a combined amount effective to kill the cell or prevent it from dividing.

### C. Immunotherapy

In one embodiment, an immunotherapy other than the CD138-specific recognition moiety (CD138-specific CAR-expressing T-cells or T-cells with CD138-specific engager molecules) is employed along with the methods of the present disclosure.

Immunotherapeutics generally rely on the use of immune effector cells and molecules to target and destroy cancer cells. The immune effector may be, for example, an antibody specific for some marker on the surface of a tumor cell. The antibody alone may serve as an effector of therapy or it may recruit other cells to actually effect cell killing. The antibody also may be conjugated to a drug or toxin (chemotherapeutic, radionuclide, ricin A chain, cholera toxin, pertussis toxin, *etc.*) and serve merely as a targeting agent. Alternatively, the effector may be a lymphocyte carrying a surface molecule that interacts, either directly or indirectly, with a tumor cell target. Various effector cells include T cells, cytotoxic T cells, NKT cells, and NK cells.

Immunotherapy could thus be used as part of a combined therapy, in conjunction with the present cell therapy. The general approach for combined therapy is discussed below. Generally, the tumor cell must bear some marker that is amenable to targeting, *i.e.,* is not present on the majority of other cells. Many tumor markers exist and any of these may be suitable for targeting in the context of the present disclosure. Common tumor markers include carcinoembryonic antigen, prostate specific antigen, urinary tumor associated antigen, fetal antigen, tyrosinase (p97), gp68, TAG-72, HMFG, Sialyl Lewis Antigen, MucA, MucB, PLAP, estrogen receptor, laminin receptor, erb B, p155, Melanoma-associated antigen (MAGE), Preferentially expressed antigen of melanoma (PRAME), survivin, CD19, CD20, CD22, k light chain, CD30, CD33, CD123, CD38, ROR1, ErbB2 ,ErbB3/4, ErbB dimers, EGFr vIII, carcinoembryonic antigen, EGP2, EGP40, mesothelin, TAG72, PSMA, NKG2D ligands, B7-H6, IL-13 receptor a2, MUC1, MUC16, CA9, GD2, GD3, HMW-MAA, CD171, Lewis Y, G250/CAIX, HLA-AI MAGE A1, HLA-A2 NY-ESO-1, PSCA, folate receptor-a, CD44v6, CD44v7/8, aᵥb₆ integrin, 8H9, NCAM, VEGF receptors, 5T4, Foetal AchR, NKG2D ligands, CD44v6, dual antigen, and universal.

### D. Genes

In yet another embodiment, the secondary treatment is a gene therapy in which a therapeutic polynucleotide is administered before, after, or at the same time as the present disclosure clinical embodiments. A variety of expression products are encompassed within the disclosure, including inducers of cellular proliferation, inhibitors of cellular proliferation, or regulators of programmed cell death.

### E. Surgery

Approximately 60% of persons with cancer will undergo surgery of some type, which includes preventative, diagnostic or staging, curative and palliative surgery. Curative surgery is a cancer treatment that may be used in conjunction with other therapies, such as the treatment of the present disclosure, chemotherapy, radiotherapy, hormonal therapy, gene therapy, immunotherapy and/or alternative therapies.

Curative surgery includes resection in which all or part of cancerous tissue is physically removed, excised, and/or destroyed. Tumor resection refers to physical removal of at least part of a tumor. In addition to tumor resection, treatment by surgery includes laser surgery, cryosurgery, electrosurgery, and miscopically controlled surgery (Mohs' surgery). It is further contemplated that the present disclosure may be used in conjunction with removal of superficial cancers, precancers, or incidental amounts of normal tissue.

Upon excision of part of all of cancerous cells, tissue, or tumor, a cavity may be formed in the body. Treatment may be accomplished by perfusion, direct injection or local application of the area with an additional anti-cancer therapy. Such treatment may be repeated, for example, every 1, 2, 3, 4, 5, 6, or 7 days, or every 1, 2, 3, 4, and 5 weeks or every 1, 2, 3, 4, 5, 6, 7, 8, 9, 10, 11, or 12 months. These treatments may be of varying dosages as well.

### F. Other Agents

It is contemplated that other agents may be used in combination with the present disclosure to improve the therapeutic efficacy of treatment. These additional agents include immunomodulatory agents, agents that affect the upregulation of cell surface receptors and GAP junctions, cytostatic and differentiation agents, inhibitors of cell adhesion, or agents that increase the sensitivity of the hyperproliferative cells to apoptotic inducers. Immunomodulatory agents include tumor necrosis factor; interferon alpha, beta, and gamma; IL-2 and other cytokines; F42K and other cytokine analogs; or MIP-1, MIP-1beta, MCP-1, RANTES, and other chemokines. It is further contemplated that the upregulation of cell surface receptors or their ligands such as Fas / Fas ligand, DR4 or DR5 / TRAIL would potentiate the apoptotic inducing abililties of the present disclosure by establishment of an autocrine or paracrine effect on hyperproliferative cells. Increases intercellular signaling by elevating the number of GAP junctions would increase the anti-hyperproliferative effects on the neighboring hyperproliferative cell population. In other embodiments, cytostatic or differentiation agents can be used in combination with the present disclosure to improve the anti-hyerproliferative efficacy of the treatments. Inhibitors of cell adhesion are contemplated to improve the efficacy of the present disclosure. Examples of cell adhesion inhibitors are focal adhesion kinase (FAKs) inhibitors and Lovastatin. It is further contemplated that other agents that increase the sensitivity of a hyperproliferative cell to apoptosis, such as the antibody c225, could be used in combination with the present disclosure to improve the treatment efficacy.

### XII. Pharmaceutical Compositions

In accordance with this disclosure, the term "pharmaceutical composition" relates to a composition for administration to an individual. In specific aspects of the disclosure, the pharmaceutical composition comprises a plurality of immune cells directed to CD138 antigen on cancer cells. In a preferred embodiment, the pharmaceutical composition comprises a composition for parenteral, transdermal, intraluminal, intra-arterial, intrathecal or intravenous administration or for direct injection into a cancer. It is in particular envisaged that said pharmaceutical composition is administered to the individual *via* infusion or injection. Administration of the suitable compositions may be effected by different ways, e.g., by intravenous, subcutaneous, intraperitoneal, intramuscular, topical or intradermal administration.

The pharmaceutical composition of the present disclosure may further comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions, *etc.* Compositions comprising such carriers can be formulated by well-known conventional methods. These pharmaceutical compositions can be administered to the subject at a suitable dose.

The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. A preferred dosage for administration might be in the range of 0.24 µg to 48 mg, preferably 0.24 µg to 24 mg, more preferably 0.24 µg to 2.4 mg, even more preferably 0.24 µg to 1.2 mg and most preferably 0.24 µg to 240 mg units per kilogram of body weight per day. Particularly preferred dosages are recited herein below. Progress can be monitored by periodic assessment. CAR-modifed T cells are adminstered via intravenous infusion. Doses can range from 1x10⁷/m² to 2x10⁸/m².

The compositions of the disclosure may be administered locally or systemically. Administration will generally be parenteral, *e.g.,* intravenous; DNA may also be administered directly to the target site, *e.g.,* by biolistic delivery to an internal or external target site or by catheter to a site in an artery. In a preferred embodiment, the pharmaceutical composition is administered subcutaneously and in an even more preferred embodiment intravenously. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishes, electrolyte replenishers (such as those based on Ringer's dextrose), and the like. Preservatives and other additives may also be present such as, for example, antimicrobials, anti-oxidants, chelating agents, and inert gases and the like. In addition, the pharmaceutical composition of the present disclosure might comprise proteinaceous carriers, like, e.g., serum albumin or immunoglobulin, preferably of human origin. It is envisaged that the pharmaceutical composition of the disclosure might comprise, in addition to the CAR constructs or nucleic acid molecules or vectors encoding the same (as described in this disclosure), further biologically active agents, depending on the intended use of the pharmaceutical composition.

Any of the compositions described herein may be comprised in a kit for treating cancers expressing CD138. In a non-limiting example, one or more CD138-directed immune cells for use in cell therapy and/or the reagents to generate one or more cells for use in cell therapy that harbors recombinant expression vectors may be comprised in a kit. The kit components are provided in suitable container means.

Some components of the kits may be packaged either in aqueous media or in lyophilized form. The container means of the kits will generally include at least one vial, test tube, flask, bottle, syringe or other container means, into which a component may be placed, and preferably, suitably aliquoted. Where there are more than one component in the kit, the kit also will generally contain a second, third or other additional container into which the additional components may be separately placed. However, various combinations of components may be comprised in a vial. The kits also will typically include a means for containing the components in close confinement for commercial sale. Such containers may include injection or blow molded plastic containers into which the desired vials are retained.

When the components of the kit are provided in one and/or more liquid solutions, the liquid solution is an aqueous solution, with a sterile aqueous solution being particularly useful. In some cases, the container means may itself be a syringe, pipette, and/or other such like apparatus, from which the formulation may be applied to an infected area of the body, injected into an animal, and/or even applied to and/or mixed with the other components of the kit.

However, the components of the kit may be provided as dried powder(s). When reagents and/or components are provided as a dry powder, the powder can be reconstituted by the addition of a suitable solvent. It is envisioned that the solvent may also be provided in another container means. The kits may also comprise a second container means for containing a sterile, pharmaceutically acceptable buffer and/or other diluent.

In particular embodiments, cells that are to be used for cell therapy are provided in a kit, and in some cases the cells are essentially the sole component of the kit. The kit may comprise reagents and materials to make the desired cell. In specific embodiments, the reagents and materials include primers for amplifying desired sequences, nucleotides, suitable buffers or buffer reagents, salt, and so forth, and in some cases the reagents include vectors and/or DNA that encodes an engager molecule as described herein and/or regulatory elements therefor.

In particular embodiments, there are one or more apparatuses in the kit suitable for extracting one or more samples from an individual. The apparatus may be a syringe, scalpel, and so forth.

In particular aspects, the kit comprises the cell therapy of the disclosure and also the chemotherapy for which the cells are immune. In some cases, the kit, in addition to the cell therapy embodiments, also includes a second cancer therapy, such as chemotherapy, hormone therapy, and/or immunotherapy, for example. The kit(s) may be tailored to a particular cancer for an individual and comprise respective second cancer therapies for the individual.

### XII. Therapeutic Uses of Host T-cells Expressing CD138 CAR

By way of illustration, cancer patients or patients susceptible to cancer or suspected of having cancer may be treated as described herein. Immune cells modified as described herein may be administered to the individual and retained for extended periods of time. The individual may receive one or more administrations of the cells. In some embodiments, the genetically modified cells are encapsulated to inhibit immune recognition and placed at the site of the tumor.

In various embodiments the expression constructs, nucleic acid sequences, vectors, host cells and/or pharmaceutical compositions comprising the same are used for the prevention, treatment or amelioration of a cancerous disease, such as a tumorous disease. In particular embodiments, the pharmaceutical composition of the present disclosure may be particularly useful in preventing, ameliorating and/or treating cancer, including cancer having solid tumors, for example.

As used herein "treatment" or "treating," includes any beneficial or desirable effect on the symptoms or pathology of a disease or pathological condition, and may include even minimal reductions in one or more measurable markers of the disease or condition being treated, *e.g.,* cancer. Treatment can involve optionally either the reduction or amelioration of symptoms of the disease or condition, or the delaying of the progression of the disease or condition. "Treatment" does not necessarily indicate complete eradication or cure of the disease or condition, or associated symptoms thereof.

As used herein, "prevent," and similar words such as "prevented," "preventing" *etc.,* indicate an approach for preventing, inhibiting, or reducing the likelihood of the occurrence or recurrence of, a disease or condition, *e.g.,* cancer. It also refers to delaying the onset or recurrence of a disease or condition or delaying the occurrence or recurrence of the symptoms of a disease or condition. As used herein, "prevention" and similar words also includes reducing the intensity, effect, symptoms and/or burden of a disease or condition prior to onset or recurrence of the disease or condition.

In particular embodiments, the present invention contemplates, in part, cells harboring expression constructs, nucleic acid molecules and/or vectors that can administered either alone or in any combination with another therapy, and in at least some aspects, together with a pharmaceutically acceptable carrier or excipient. In certain embodiments, prior to administration of the cells, said nucleic acid molecules or vectors may be stably integrated into the genome of the cells. In specific embodiments, viral vectors may be used that are specific for certain cells or tissues and persist in said cells. Suitable pharmaceutical carriers and excipients are well known in the art. The compositions prepared according to the disclosure can be used for the prevention or treatment or delaying the above identified diseases.

Furthermore, the disclosure relates to a method for the prevention, treatment or amelioration of a cancerous (including tumorous) disease comprising the step of administering to a subject in need thereof an effective amount of cells harboring an antigen recognition moiety molecule and a chemotherapy resistance molecule, nucleic acid sequence that encodes them, vector(s) that encodes them, as contemplated herein and/or produced by a process as contemplated herein.

Possible indications for administration of the composition(s) of the exemplary modified immune cells are cancerous diseases, including tumorous diseases, including breast, prostate, lung, and colon cancers or epithelial cancers/carcinomas such as MM, breast cancer, colon cancer, prostate cancer, head and neck cancer, skin cancer, cancers of the genito-urinary tract, *e.g.* ovarian cancer, endometrial cancer, cervix cancer and kidney cancer, lung cancer, gastric cancer, cancer of the small intestine, liver cancer, pancreas cancer, gall bladder cancer, cancers of the bile duct, esophagus cancer, cancer of the salivary glands and cancer of the thyroid gland. Exemplary indications for administration of the composition(s) of cells are cancerous diseases, including any malignancies that express CD138, for example. In addition, it includes malignancies that aberrantly express other tumor antigens and those may also be targeted. The administration of the composition(s) of the disclosure is useful for all stages and types of cancer, including for minimal residual disease, early cancer, advanced cancer, and/or metastatic cancer and/or refractory cancer, for example.

The disclosure further encompasses co-administration protocols with other compounds, e.g. bispecific antibody constructs, targeted toxins or other compounds, which act *via* immune cells. The clinical regimen for co-administration of the inventive compound(s) may encompass co-administration at the same time, before and/or after the administration of the other component. Particular combination therapies include chemotherapy, radiation, surgery, hormone therapy, or other types of immunotherapy.

Embodiments relate to a kit comprising one or more immune cells as described herein, a nucleic acid sequence as described herein, a vector as described herein and/or a host as described herein. It is also contemplated that the kit of this disclosure comprises a pharmaceutical composition as described herein above, either alone or in combination with further medicaments to be administered to an individual in need of medical treatment or intervention.

The CTLs that have been modified with the construct(s) are then grown in culture under selective conditions and cells that are selected as having the construct may then be expanded and further analyzed, using, for example; the polymerase chain reaction for determining the presence of the construct(s) in the host cells. Once the modified host cells have been identified, they may then be used as planned, *e.g*., expanded in culture or introduced into a host organism.

Depending upon the nature of the cells, the cells may be introduced into a host organism, *e.g*., a mammal, in a wide variety of ways. The cells may be introduced at the site of the tumor, in specific embodiments, although in alternative embodiments the cells hone to the cancer or are modified to hone to the cancer. The number of cells that are employed will depend upon a number of circumstances, the purpose for the introduction, the lifetime of the cells, the protocol to be used, for example, the number of administrations, the ability of the cells to multiply, the stability of the recombinant construct, and the like. The cells may be applied as a dispersion, generally being injected at or near the site of interest. The cells may be in a physiologically-acceptable medium.

The DNA introduction need not result in integration in every case. In some situations, transient maintenance of the DNA introduced may be sufficient. In this way, one could have a short term effect, where cells could be introduced into the host and then turned on after a predetermined time, for example, after the cells have been able to home to a particular site.

The cells may be administered as desired. Depending upon the response desired, the manner of administration, the life of the cells, the number of cells present, various protocols may be employed. The number of administrations will depend upon the factors described above at least in part.

It should be appreciated that the system is subject to many variables, such as the cellular response to the ligand, the efficiency of expression and, as appropriate, the level of secretion, the activity of the expression product, the particular need of the patient, which may vary with time and circumstances, the rate of loss of the cellular activity as a result of loss of cells or expression activity of individual cells, and the like. Therefore, it is expected that for each individual patient, even if there were universal cells which could be administered to the population at large, each patient would be monitored for the proper dosage for the individual, and such practices of monitoring a patient are routine in the art.

### EXAMPLES

The following examples are included to demonstrate preferred embodiments of the disclosure. It should be appreciated by those of skill in the art that the techniques disclosed in the examples that follow represent techniques discovered by the inventor to function well in the practice of the disclosure, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the disclosure.

### EXAMPLE 1

### INITIAL STUDIES

### XII. Initial Studies/Preliminary Data

Clinical trials have been developed that target CD19, the κ-light chain of human immunoglobulins and CD30 in lymphomas using CAR-based technology. The strategies are under clinical investigation, and they recently reported the first group of patients treated with CAR.CD19. In embodiments of the present disclosure, CAR technology is used to target an exemplary hematological disorder that has not yet been selected for treatment by this approach.

**CAR.CD138-redirected T cells target CD138+ malignant PC.** The inventors cloned a CD138-specific single chain (scFv) in frame with the IgG1 hinge-CH2CH3 regions, CD28 endodomain and ζ-chain (2nd generation CAR) as previously described. FIG. 1 illustrates the expression of the CAR.CD138 in activated T lymphocytes and their specific killing of CD138+ MM cell lines (U266 and RPMI) and primary neoplastic PC.

**CAR.CD138+ T cells control MM growth *in vivo.*** To evaluate the antitumor effects of CAR-redirected T cells *in vivo,* the inventors established a xenograft model in SCID mice, and used a bioluminescence system to track MM growth. For these experiments, mice were injected intravenously (i.v) with U266 cells labeled with Firefly luciferase (FFLuc) (0.5x10⁶ cells). When tumor cells were consistently detectable by luminescence, mice were injected i.v. with control and CAR+ T cells (10⁷ cells/mouse) without exogenous cytokines. As shown in FIG. 2, CAR+ T cells provide control of MM growth.

Hypoxia inducible expression of CAR.CD138. To generate the hypoxia inducible CAR.CD138, the inventors inverted the orientation of the CAR.CD138 cassette into the SFG retroviral backbone, and included the 6 repeats of the HRE fused with a minimal CMV promoter (Clontech) at its 3' end. FIG. 3 illustrates the expression and function of the hypoxia inducible CAR.CD138 (hCAR.CD138) in activated T lymphocytes under normoxic and hypoxia (1% O₂ tension).

Overall these initial studies indicate the practicality of the disclosure. Importantly, the CAR.CD138 expressed under the control of HRE retains full anti-MM function.

### EXAMPLE 2

### EXEMPLARY METHODS

Several methods used routinely are described fully in previous publications (9, 25).

In embodiments of the disclosure, there is exploitation of the hypoxic nature of MM BM microenvironment by expressing the CAR.CD138 under the inducible control of hypoxia-responsive elements (HRE). One can also define the most advantageous immune elements for costimulation of CAR T cells in a hypoxic environment both *in vitro* and *in vivo* in a Xenogenic mouse model. Finally, to further increase the safety of the proposed approach, the skilled artisan can incorporate within a construct a previously validated suicide gene based on inducible caspase9 (iC9).

The initial studies (FIGS. 1 and 2) clearly indicate that T cells that constitutively express CAR.CD138 have anti MM effects both *in vitro* and *in vivo* in xenotransplant mouse model. As shown in FIG. 3, hCAR.CD138 is significantly upregulated when T cells are exposed to hypoxia, and these cells retain anti-MM activity. The CAR.CD138 in these studies encodes the CD28 costimulatory endodomain. One can compare *in vitro* the effects of costimulatory endodomains, including 4-1BB, and their combination in hypoxia. One can measure *in vitro* proliferation, cytokine release and cytotoxic activity after serial antigen stimulation and anti-tumor activity *in vivo* in a mouse model (FIG. 2). In addition, because dim CAR expression is still observed even under normoxia (FIG. 3), an insulator between the HRE and the 3' end LTR of the vector can be included to further reduce expression. Upon functional expression of the hCAR.CD138 in BM hypoxia, T cells with self-renewal capacity may escape the BM environment and recirculate, creating potential toxicity. Because it takes 48-72 hours for the T cells to downregulate CAR expression when transferred from hypoxia to normoxia (in at least some embodiments), one can co-express a suicide gene based on the inducible caspase9 (iC9), under a constitutive promoter so they can be rapidly eliminated if they cause toxicity. The iC9 gene can be accommodated in the retroviral vector described in FIG. 3 in opposite orientation under the constitutive control of the 5' LTR. The functionality of the vector *in vitro* and *in vivo* is evaluated.

In embodiments of the disclosure, a useful vector allows predominant and functional expression of the CAR.CD138 in hypoxia both *in vitro* and *in vivo.* In addition, the inclusion of the suicide gene is anticipated to eliminate the cells in case of toxicity. If the CAR expression in hypoxia is functionally insufficient, one may attribute that to the size of the cassette that includes the insulator (total size 3,5 kb) and can transfer the cassette in a lentivirus vector that has increased cargo capacity, as compared to retroviral vectors. A retroviral approach may be employed because the manufacturing of these vectors is robust and highly reproducible, although in some cases lentivirus is utilized.

In an embodiment of the disclosure, there is a method that encompasses manufacturing the clinical grade retroviral vector and CAR-modified T-cell lines from MM patients and infusing them into patients with relapsed MM. The skilled artisan can evaluate the safety of the procedure and if toxicity occurs, one can administer the dimerizer drug to activate the iC9 safety gene *in vivo.* One can also assess whether T-cell infusions provide disease control in patients with detectable disease.

A stable retroviral producer cell line encoding the hCAR.CD138 and constitutive iC9 is produced. As previously described, PG13 packaging may be employed. The detailed procedures for preparing the producer cell line are provided in the core description. The clinical grade supernatant obtained from this packaging may be used to transduce T cells from PB samples obtained from MM patients.

One can evaluate the effects of increasing doses of T lymphocytes expressing the hCAR.CD138 and iC9 in patients with relapsed/refractory MM (in contrast other embodiments, in one can treat patients responsive to standard first line therapies). One can initially evaluate a single intravenous dose of CAR-T cells at three different dose levels. T cells are administered after treatment with bendamustine because this drug has some anti-MM activity and likely creates a favorable environment for the expansion of adoptively transferred CAR T-cells.

A stable producer line with a titer > 10⁶ per mL may be developed. The T-cell transduction efficiency may range from 30% to 40% in hypoxia (FIG. 3). Redirected T cells can eliminate CD138+ targets in coculture experiments in hypoxia and can be rapidly eliminated (>90% of transduced cells) within 24hrs of *in vitro* exposure to AP1903 (20-50 nM), the small molecule dimerizer that activates the iC9 suicide gene.

In embodiments of the disclosure, the fate of the infused CAR-T cells is characterized by measuring their *in vivo* survival, and the subsequent effects of the dimerizing drug on these cells *in vitro* and *in vivo* can be determined. One can also compare the accumulation of these cells in the BM and peripheral blood, the differential expression of the CAR by T cells in each environment, and their related ability to kill tumor cells.

Persistence and expansion of transferred T cells are estimated using immunophenotype and real-time quantitative PCR assay (Q-PCR) in DNA extracted from PB collected at different time points after T-cell infusions. BM samples are collected at week 6 as part of the clinical evaluation of these patients. Plasma samples are collected and snap frozen at intervals to detect IL-2, IL6, IFNγ and TNFα released by T cells *in vivo.* One can also seek clinical evidence of antitumor activity by monitoring paraprotein levels and PC BM infiltration at 6, 12 and 24 weeks, for example. Patients are closely monitored for the occurrence of toxicity according to NCI criteria. In the case of grade III-IV toxicity, patients are treated with AP1903 to activate the iC9 gene as previously reported. The iC9 effects are quantified by measuring the loss of CAR+ circulating cells and by changes in the iC9 transgene by QPCR.

The molecular signal of the CAR (which is hypoxia independent) is detected both in PB and BM samples by week 6, even though there may be higher signal in the BM because of the accumulation of CAR T cells. The expression of the CAR by flow cytometry in T cells (which is hypoxia dependent, as it detects protein synthesis) is anticipated to be much higher in BM as compared to PB because MM BM is hypoxic. If equal numbers of T cells are phenotypically CAR positive in both PB and BM, this reflects the recirculation of hypoxia activated T cells without the anticipated down regulation of CAR that is expected when the cells return to a normoxic environment, in specific embodiments. One can determine whether this continuing expression is associated with toxicity, and if it is, one can determine whether it can be abrogated by administration of AP1903 that activates the iC9 suicide gene. The activation of iC9 may eliminate >90% hCAR.CD138T within 3 hours of AP1903 administration. If side effects continue to increase despite the administration of AP1903 one can administer additional doses of drug and high dose steroids. In specific embodiments, the infusion of hCAR.CD138 T cells produces significant reduction of tumor. In the event that a single dose of T cells is insufficient to induce complete remission, one can administer additional doses of T cells.

In specific embodiments, the CAR.CD138 is engrafted onto third party EBV-specific CTLs, in order to make "off the shelf' products for patients with MM, an approach of particular value after the non-myeloablative allogeneic stem cell transplants that may be used for older MM patients with severe disease. In some embodiments there is targeting of other tumor associated antigens (MAGE, PRAME, Survivin) using TCR's directed to MM TAA. Inclusion of a third specificity mediated by a CAR rather than a TCR may significantly reduce tumor escape due to the loss of antigens or HLA molecule expression/antigen processing, in some embodiments. One can express the CD138 scFv as a secretable "engager" protein.

### EXAMPLE 3

### STUDY OF T CELLS EXPRESSING CD138-SPECIFIC CAR FOR ADVANCE PLASMA CELL DYSCRASIAS

In embodiments of the disclosure, one can evaluate the safety of escalating doses of autologous or syngeneic activated peripheral blood T lymphocytes (ATLs) genetically modified to express (a) an artificial T-cell receptor (chimeric antigen receptor or CAR) targeting the CD138 molecule (CD138.CAR) under an hypoxia-dependent promoter, and (b) a inducible caspase-9 (iC9) suicide protein under a constitutively active promoter. Specific embodiments of methods of the disclosure include (1) measuring the survival and function of these CD138.CAR-ATLs *in vivo;* (2) quantifying the anti-tumor effects of CD138.CAR-ATLs in patients with refractory plasma cell dyscrasias, with clinical responses assessed by the modified International Myeloma Working Group (IMWG) Uniform Response criteria; and (3) evaluating the efficacy of the administration of AP1903, a dimerizer used to activate the suicide gene, by measuring disappearance of transgene positive cells from peripheral blood, should toxicity occur.

In at least some cases for an initial study, there is exemplary eligibility criteria for T cell treatment. (1) refractory/relapsed plasma cell dyscrasia (or newly diagnosed if patients unable to receive or complete standard therapy); (2) life expectancy of at least 12 weeks; (3) adequate organ function; (4) available autologous transduced peripheral blood T cells with >20% expression of CD138.CAR/iC9 determined by flow-cytometry; (5) signed informed consent; (6) no history of hypersensitivity reactions to murine protein-containing products.

Example of treatment plan. Administration of bendamustine. Patients receive bendamustine (45 mg/m² daily for 2 days, for example) to generate lymphodepletion and favor engraftment of the infused CAR+ T cells.

Bendamustine may also have limited anti-myeloma effects. T cell administration. T cells are infused on day 4-7 after bendamustine treatment to maximize their exposure to the developing milieu of regenerative cytokines including IL-7 and IL-15. Three dose levels are evaluated using the modified continual reassessment method and cohorts of size two are enrolled at each dose level. Each patient receives one injection according to the following dosing plan: Group 1, 2×10⁷ cells/m₂; Group 2, 1×10⁸ cells/m²; Group 3, 2×10⁸ cells/m². In the absence of direct toxicity, one can obtain permission to administer sequential doses of T cells, a strategy followed in exemplary previous studies.

**Monitoring clinical and biological parameters.** One can monitor certain parameters and include a history and physical examination and routine laboratory investigations performed pre- and at 4 hours and 1, 2, 4, 6 weeks post T-cell infusion. One can also monitor the persistence of CAR+ T cells by a specific Q-PCR assay as described for other studies designed to detect retroviral integrants. Detection of replication-competent retroviruses (RCRs) and of retroviral integrant clonality are performed as per FDA guidelines. Disease burden assessment, including BM, pre and 6 wks post-treatment is utilized

**Dose-limiting toxicity (DLT).** DLT may be defined as any of the following that may be considered possibly, probably, or definitely related to the modified T cells: (1) any Grade 3 or Grade 4 nonhematologic toxicity; (2) any Grade 4 hematologic DLT (cytopenias are expected with bendamustine and will not be graded as DLT; patients with extensive bone marrow involvement are not evaluable for hematological DLT). Should non-hematologic DLT occur, patients will be treated with a single dose of dimerizer drug (0.4 mg/kg) and the effects on circulating gene modified T cells studied. If there is no decreasing toxicity within 48 hr, the protocol will allow for up to 3 additional doses of dimerizer drug in combination with steroids (1 mg/kg daily of methylprednisolone).

**Termination of study.** The trial may end when a minimum of 10 patients are treated, with 6 patients accrued at the current dose, if there is no DLT, or when the predictive probability of DLT is >20%. A maximum of 18 patients may be accrued. Toxicity is evaluated using NCI criteria (CTCAE). A 6-wk period constitutes a treatment course and serves as the basis for evaluating for DLT, in specific embodiments

### EXAMPLE 4

### CD138 CAR T-CELLS ARE EFFECTIVE IN VITRO AND IN VIVO

The present examples illustrates by *in vitro* and *in vivo* measures that the exemplary CD138-specific CAR T-cells are effective against tumor cells.

FIG. 4 demonstrates that CAR.CD138 can be efficiently and stably expressed in T cells from both healthy donors and multiple myeloma (MM) samples. As shown in FIG. 4D, both control and transduced T cells from healthy donors contained a balanced proportion of CD3⁺CD8⁺ T cells (57%±26% and 54%±14%) and CD3⁺CD4⁺ T cells (35%±17% and 37%±13%), while T cells from MM patients were more skewed to contain CD8⁺ cells (80%±10%). Transduced T cells from healthy donors and MM patients contained a proportion of memory and effector memory cells (CD45RO⁺: 82%±16% and 79%±9%, respectively; CD62L⁺=51%±17% and 42%±14%, respectively) compatible with the *ex vivo* expansion procedure.

In FIG. 5, CAR.CD138⁺ T cells target CD138⁺ tumor cell lines. T cells from healthy donors expressing CAR.CD138 lysed selected CD138⁺ MM-derived cell lines at a significantly higher rate than control T cells in a standard 51Cr release assay (FIGS. 5A, 5D). Similar pattern of killing was observed when transduced T cells were generated form MM patients (FIG. 5B). In contrast, CAR.CD138⁺ T cells had negligible activity against particular CD138⁻ targets (FIGS. 5A, 5B, 5D) or control T cells (FIG. 5C).

FIG. 6 demonstrates that CAR.CD138⁺ T cells eliminate CD138⁺ tumor cells in co-culture experiments. To evaluate the long-term ability of CAR.CD138⁺ to eliminate CD138⁺ tumor cells, CAR⁺ or control T cells were co-cultured with CD138⁺ tumor cells or CD138⁻ tumor cells in the absence of exogenenous cytokines (FIG. 6A); residual tumor cells were enumerated after 5-7 days by FACS analysis. In the presence of CAR⁺ T cells there was complete elimination of CD138⁺ tumors, while tumor cells overgrew in cultures with control T cells.

CAR.CD138⁺ T cells show a Th1 profile in response to tumor cells (FIG. 7). To evaluate the cytokine profile of CAR.CD138⁺, CAR⁺ or control T cells were co-cultured with CD138⁺ or CD138⁻ tumor cells. Culture supernatants were collected after 24 hours and analyzed for the presence of particular Th1 and Th1 cytokines.

CAR.CD138⁺ T cells target putative cancer stem cells (FIG. 8). To ensure that the approach also targets putative cancer stem cells, expression of CD138 by SP cells contained in the RPMI-8266 tumor cell cells was studied and then it was monitored if this subset could also be effectively eliminated by CAR⁺ T cells. In co-cultures with control T cells, not only RPMI-8266 cells were still present, but also an average of 6% of SP cells was still present (FIG. 8A, 8B). In contrast, in cultures with CAR⁺ T cells, RPMI cells were significantly reduced and no SP cells were detectable (FIG. 8A, 8B). To further confirm this capability, SP cells were directly sorted from the RPMI cell line and cultured with control or CAR⁺ T cells (FIG. 8C). SP cells sorted cells were completely eliminated only in the presence of transduced T cells.

FIG. 9 shows that CAR.CD138⁺ T cells target primary myeloma cells. CAR⁺ T cells generated from healthy donors successfully eliminated CD138 selected tumor cells from MM patients in contrast to control T cells (<80% fold reduction) (FIG. 9A). In FIG. 9B, similarly autologous CAR⁺ T cells eliminated primary MM cells as compared to control T cells, and cytokine profile in these experiments was consistent with Th1 (FIG. 9C).

FIG. 10 demonstrates that CAR.CD138⁺ T cells have antitumor activity *in vivo.* NSG mice received intravenous administration of 4x10⁶ FireFlyLuciferase labeled OPM-2 cells, followed by 3 i.v. infusions with CAR.CD138⁺ T cell infusions (1x10⁷). Cioluminescent imaging (BLI) was performed starting on day 23 to monitor tumor growth. FIG. 10A shows average photons/sec/cm²/sr per mouse, determined by BLI, comparing mice treated with control T cells or CAR.CD138⁺ T cells. Summary of 3 independent experiments. FIG. 10B illustrates a Kaplan-Meier survival curve of mice treated with CAR.CD138⁺ T cells or control T cells (p<0.01).

In FIG. 11, there is generation and function of hypoxia inducible CAR.CD138 (HRE.CAR.CD138). FIG. 11A provides a schematic representation of the HRE.CAR.CD138 encoded in a retroviral vector. Control, constitutive CAR.CD138⁺ or HRE.CAR.CD138⁺ T cells were co-cultured with the CD138⁺ targets in normoxia or hypoxia (FIG. 11B). After 4 days of culture, cells were collected and stained with CD3 and CD138 to evaluate the growth of tumor cells. Expression of CAR on T cells was also evaluated. HRE.CAR.CD138⁺ T cells eliminated the tumor cells in hypoxic conditions. (FIG. 11C) Control, constitutive CAR.CD138⁺ or HRE.CAR.CD138⁺ T cells were labeled and co-cultured with the CD138⁺ targets in normoxia or hypoxia. After 4 days of culture, cells were collected, stained with CD3 and dilution of CSFE measured by flow cytometry. HRE.CAR.CD138⁺ T cells proliferated in hypoxic conditions.

Although the present invention and its advantages have been described in detail, it should be understood that various changes, substitutions and alterations can be made herein without departing from the spirit and scope of the invention as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the disclosure of the present invention, processes, machines, manufacture, compositions of matter, means, methods, or steps, presently existing or later to be developed that perform substantially the same function or achieve substantially the same result as the corresponding embodiments described herein may be utilized according to the present invention. Accordingly, the appended claims are intended to include within their scope such processes, machines, manufacture, compositions of matter, means, methods, or steps.

## Claims

1. A method of producing an immune cell expressing a CD138-specific chimeric antigen receptor (CAR), the method comprising transfecting *ex vivo* an immune cell with an expression vector that encodes the CAR and one or more hypoxia- responsive regulatory elements functionally related thereto.

2. The method of claim 1, wherein said expression vector further comprises an inducible suicide gene.

3. The method of claim 1, wherein the CD138-specific CAR comprises an IgG1 hinge region.

4. The method of claim 1, wherein the CD138-specific CAR comprises an intracellular signaling domain from CD28, OX40, 4-1BB, ICOS, or a combination thereof.

5. The method of claim 2 wherein the suicide gene encodes caspase 9, herpes simplex virus, thymidine kinase (HSV-tk), cytosine deaminase (CD) or cytochrome P450.

6. The method of claim 1 or claim 2, wherein the hypoxia- responsive regulatory element comprises a VEGF hypoxia-responsive regulatory element, an α1B-adrenergic receptor hypoxia-responsive regulatory element, fatty acid synthase hypoxia- responsive regulatory element, or a combination thereof.

7. The method of claim 1, wherein the immune cell is autologous to an individual having a cancer that expresses CD138.

8. The method of claim 7, wherein said cancer is a B-cell lineage hematologic malignancy.

9. The method of claim 7, wherein said cancer is multiple myeloma.

10. The method of claim 1, wherein the immune cell is a cytotoxic T lymphocyte (CTL), natural killer cell, or natural killer T cell.

11. The method of any of claims 1-10, wherein the immune cell comprises at least one other CAR specific for an antigen other than CD138, optionally wherein the CAR is specific for an antigen selected from the group consisting of Melanoma-associated antigen (MAGE), Preferentially expressed antigen of melanoma (PRAME), survivin, CD19, CD20, CD22, k light chain, CD30, CD33, CD123, CD38, ROR1, ErbB2, ErbB3/4, ErbB dimers, EGFr vIII, carcinoembryonic antigen, EGP2, EGP40, mesothelin, TAG72, PSMA, NKG2D ligands, B7-H6, IL-13 receptor a2, MUC1, MUC16, CA9, GD2, GD3, HMW-MAA, CD171, Lewis Y, G250/CAIX, HLA-AI MAGE Al, HLA-A2 NY-ESO-1, PSCA, folate receptor-a, CD44v6, CD44v7/8, avb6 integrin, 8H9, NCAM, VEGF receptors, 5T4, Foetal AchR, NKG2D ligands, CD44v6, dual antigen, and universal.

## Patentansprüche

1. Verfahren zum Herstellen einer Immunzelle, die einen CD138-spezifischen chimären Antigen-Rezeptor (CAR) exprimiert, wobei das Verfahren das Transfizieren einer Immunzelle *ex vivo* mit einem Expressionsvektor umfasst, der für den CAR und eine oder mehrere Hypoxie-ansprechende regulatorische Elemente kodiert, die damit verwandt sind.

2. Verfahren nach Anspruch 1, wobei der Expressionsvektor ferner ein induzierbares Selbstmord-Gen umfasst.

3. Verfahren nach Anspruch 1, wobei der CD138-spezifische CAR eine IgG1-Gelenkregion umfasst.

4. Verfahren nach Anspruch 1, wobei der CD138-spezifische CAR eine intrazelluläre Signalisierungsdomäne von CD28, OX40, 4-1BB, ICOS oder einer Kombination davon umfasst.

5. Verfahren nach Anspruch 2, wobei das Selbstmord-Gen für Caspase-9, HerpesSimplex-Virus, Thymindin-Kinase (HSV-tk), Cytosin-Deaminase (CD) oder Cytochrom-P450 kodiert.

6. Verfahren nach Anspruch 1 oder 2, wobei das Hypoxie-ansprechende regulatorische Element ein Hypoxie-ansprechendes regulatorisches VEGF-Element, ein Hypoxie-ansprechendes regulatorisches Element des α1B-andrenergenen Rezeptors, ein Hypoxie-ansprechendes regulatorisches Element der Fettsäure-Synthase oder eine Kombination davon umfasst.

7. Verfahren nach Anspruch 1, wobei die Immunzelle autolog zu einem Individuum mit Krebs ist, die CD138 exprimiert.

8. Verfahren nach Anspruch 7, wobei der Krebs ein hämatologisches Malignom der B-Zellen Abstammung ist.

9. Verfahren nach Anspruch 7, wobei der Krebs ein multiples Myelom ist.

10. Verfahren nach Anspruch 1, wobei die Immunzelle ein zytotoxischer T-Lymphozyt (CTL), eine natürliche Killerzelle oder eine natürlich Killer-T-Zelle ist.

11. Verfahren nach einem der Ansprüche 1 bis 10, wobei die Immunzelle wenigstens einen anderen CAR umfasst, der spezifisch für ein anderes Antigen als CD138 ist, optional wobei der CAR für ein Antigen spezifisch ist, das aus der Gruppe ausgewählt ist, die aus einem mit einem Melanom assoziiertem Antigen (MAGE), einem vorzugsweise exprimierten Antigen eines Melanoms (PRAME), Survivin, CD19, CD20, CD22, k leichter Kette, CD30, CD33, CD123, CD38, ROR1, ErbB2, ErbB3/4, ErbB-Dimeren, EGFr vIII, einem carcinoembryonischen Antigen, EGP2, EGP40, Mesothelin, TAG72, PSMA, NKG2D-Liganden, B7-H6, IL-13-Repeptor a2, MUC1, MUC16, CA9, GD2, GD3, HMW-MAA, CD171, Lewis Y, G250/CAIX, HLA-AI MAGE A1, HLA-A2 NY-ESO-1, PSCA, Folat-Rezeptor-a, CD44v6, CD44v7/8, avb6-Integrin, 8H9, NCAM, VEGF-Rezeptoren, 5T4, Fötal AchR, NKG2D-Liganden, CD44v6, einem dualen Antigen und universell besteht.

## Revendications

1. Procédé de production d'une cellule immunitaire exprimant un récepteur d'antigène chimérique (CAR) spécifique au CD138, le procédé comprenant la transfection *ex vivo* d'une cellule immunitaire avec un vecteur d'expression qui code pour le CAR et un ou plusieurs éléments régulateurs sensibles à l'hypoxie fonctionnellement liés à celle-ci.

2. Procédé selon la revendication 1, dans lequel ledit vecteur d'expression comprend en outre un gène suicide inductible.

3. Procédé selon la revendication 1, dans lequel le CAR spécifique au CD138 comprend une région charnière de l'IgG1.

4. Procédé selon la revendication 1, dans lequel le CAR spécifique au CD138 comprend un domaine de signalisation intracellulaire de CD28, OX40, 4-1BB, ICOS, ou une combinaison de ceux-ci.

5. Procédé selon la revendication 2, dans lequel le gène suicide code pour la caspase 9, le virus de l'herpès simplex, la thymidine kinase (HSV-tk), la cytosine désaminase (CD) ou le cytochrome P450.

6. Procédé selon la revendication 1 ou la revendication 2, dans lequel l'élément régulateur sensible à l'hypoxie comprend un élément régulateur sensible à l'hypoxie VEGF, un élément régulateur sensible à l'hypoxie de récepteur alB-adrénergique, un élément régulateur sensible à l'hypoxie d'acides gras synthase, ou une combinaison de ceux-ci.

7. Procédé selon la revendication 1, dans lequel la cellule immunitaire est autologue à un individu ayant un cancer qui exprime le CD138.

8. Procédé selon la revendication 7, dans lequel ledit cancer est une malignité hématologique de lignée de cellules B.

9. Procédé selon la revendication 7, dans lequel ledit cancer est un myélome multiple.

10. Procédé selon la revendication 1, dans lequel la cellule immunitaire est un lymphocyte T cytotoxique (CTL), une cellule tueuse naturelle ou une cellule T tueuse naturelle.

11. Procédé selon l'une quelconque des revendications 1 à 10, dans lequel la cellule immunitaire comprend au moins un autre CAR spécifique à un antigène autre que CD138, éventuellement dans lequel le CAR est spécifique à un antigène sélectionné dans le groupe constitué par l'antigène associé au mélanome (MAGE), l'antigène de mélanome préférentiellement exprimé (PRAME), la survivine, CD19, CD20, CD22, la chaîne légère k, CD30, CD33, CD123, CD38, ROR1, ErbB2, ErbB3 / 4, les dimères ErbB, EGFr vIII, l'antigène carcinoembryonnaire, EGP2, EGP40, la mésothéline, TAG72, PSMA, les ligands NKG2D, B7-H6, le récepteur IL-13 a2, MUC1, MUC16, CA9, GD2, GD3, HMW-MAA, CD171, Lewis Y, G250 / CAIX, HLA-AI MAGE A1, HLA-A2 NY-ESO-1, PSCA, le récepteur de folate a, CD44v6, CD44v7 / 8, l'intégrine avb6, 8H9, NCAM, les récepteurs VEGF, 5T4, AchR fœtal, les ligands NKG2D, CD44v6, les double-antigène, et l'universel.
